# EUROPEAN PATENT SPECIFICATION

(11) **EP 3 289 355 B1**
(45) Date of publication and mention of the grant of the patent: **02.07.2025**
(21) Application number: 16787315.7
(22) Date of filing: 02.05.2016
(51) Int. Cl.: G01N 33/68, G01N 33/53, C07K 16/44

(54) **METHODS FOR DETECTING RENAL DISEASE**
VERFAHREN FÜR DEN NACHWEIS VON NIERENERKRANKUNGEN
PROCÉDÉS DE DÉTECTION D'UNE MALADIE RÉNALE

(30) Priority: 30.04.2015 US 201562155158 P
(43) Date of publication of application: 07.03.2018
(73) Proprietor: IDEXX Laboratories, Inc., Westbrook, ME 04092 (US)
(72) Inventor: YERRAMILLI, Mahalakshmi, Falmouth, ME 04105 (US); OBARE, Edward, Westbrook, ME 04092 (US); QUINN, John, Joseph, Falmouth, ME 04105 (US); YERRAMILLI, Murthy, VSN, Falmouth, ME 04105 (US)
(74) Representative: Hoffmann Eitle
(86) International application number: PCT/US2016/030478
(87) International publication number: WO 2016/176691

(56) References cited:
- WO-A1-2011/130385
- WO-A1-2013/115283
- WO-A1-2015/035155
- WO-A2-2013/086365
- WO-A2-2013/086365
- US-A1- 2007 042 497
- US-A1- 2012 129 265
- GEJYO ET AL: "Elevation of Serum Levels of Beta-Aminoisobutyric Acid in Uremic Patients and the Toxicity of the Amino Acid", CLINICAL NEPHROLOGY, DUSTRI VERLAG, NUENCHEN-DEISENHOFEN, DE, vol. 8, no. 6, 1 January 1977 (1977-01-01), pages 520 - 525, XP009507042, ISSN: 0301-0430
- EVERED D F ET AL: "Infrequency of urinary excretion of beta-aminoisobutyric acid by healthy humans", CLINICA CHIMICA ACTA, ELSEVIER BV, AMSTERDAM, NL, vol. 84, no. 3, 15 March 1978 (1978-03-15), pages 339 - 346, XP025200043, ISSN: 0009-8981, [retrieved on 19780315], DOI: 10.1016/0009-8981(78)90250-4
- KITTEL ANJA ET AL: "In vivoevidence that Agxt2 can regulate plasma levels of dimethylarginines in mice", BIOCHEMICAL AND BIOPHYSICAL RESEARCH COMMUNICATIONS, ELSEVIER, AMSTERDAM, NL, vol. 430, no. 1, 12 November 2012 (2012-11-12), pages 84 - 89, XP028962866, ISSN: 0006-291X, DOI: 10.1016/J.BBRC.2012.11.008
- "Principles and practice of immunoassay", 1 January 1991, MACMILLAN PUBLISHERS LTD, New York, N.Y., ISBN: 978-1-349-11234-0, article JACKY BURRIN ET AL: "Production of antibodies", pages: 20 - 26, XP055704384
- ALAUDDIN M M ET AL: "@a-Alkylation of Amino Acid Derivatives: Synthesis and Chiral Resolution of [^1^1C]@b-Aminoisobutyric Acid", NUCLEAR MEDICINE AND BIOLOGY, ELSEVIER, NY, US, vol. 24, no. 8, 1 November 1997 (1997-11-01), pages 771 - 775, XP004097018, ISSN: 0969-8051, DOI: 10.1016/S0969-8051(97)00121-2
- GEJYO, F ET AL.: "Elevation of Serum Levels of Beta-Aminoisobutyric Acid in Uremic Patients and the Toxicity of the Amino Acid.", CLINICAL NEPHROLOGY., vol. 8, no. 6, 1977, pages 520 - 525, XP 009507042
- ZEIS, PM ET AL.: "DNA Degradation in the Kidney of Folic Acid-Treated Guinea Pigs.", CYTOBIOS., vol. 102, no. 400, 2000, pages 107 - 113, XP009507047
- WORCESTER, EM ET AL.: "Evidence That Serum Calcium Oxalate Supersaturation is a Consequence of Oxalate Retention in Patients with Chronic Renal Failure.", JOURNAL OF CLINICAL INVESTIGATION., vol. 77, no. 6, June 1986 (1986-06-01), pages 1888 - 1896, XP 055327612
- OTTERSEN, OP ET AL.: "Evaluation of the Immunocytochemical Method for Amino Acids.", MEDICAL BLOLOGY, vol. 84, no. 2-3, pages 147 - 150, XP 009507048
- KITTEL, A ET AL.: "Alanine-glyoxylate aminotransferase 2 (AGXT2) Polymorphisms Have Considerable Impact on Methylarginine and Beta-aminoisobutyrate Metabolism in Healthy Volunteers.", PLOS ONE., vol. 9, no. 2, 2014, XP 055327616
- SUHRE, K ET AL.: "A Genome-Wide Association Study of Metabolic Traits in Human Urine.", NATURE GENETICS., vol. 43, no. 6, 2011, pages 566 - 569, XP 055006593

## Description

### BACKGROUND

### Field

The disclosure generally relates to the determination of renal function. More particularly, the disclosure relates to methods for diagnosing and determining the progression of kidney disease.

### Related Art

It is important to be able to measure renal function quickly and accurately. For example, the dosing of drugs must be adapted for patients with renal insufficiency. Thus, making an accurate assessment of renal function is a requirement in clinical medicine. However, the diagnosis of renal insufficiency is hindered by the lack of reliable markers and/or available diagnostic tests. In clinical practice, serum creatinine is typically used to assess renal function. The use of serum creatinine can, however, suffer from imprecision, as data can be subject to a relatively high degree of variability. In addition, it is known that up to 75% of kidney function may be lost by the time that creatinine is Gejyo et al. (Clinical Nephrology. 1977. 8:520-525) describe markers, amongst these β-aminoisobutyric acid by chromatography in order to determine glomerulonephritis in patient samples. WO2013/086365 discloses diagnosis of early nephropathy by measuring the concentration of various amino acids, amongst these BAIB, in blood samples of subjects to be tested versus healthy controls using mass spectrometry.

Accordingly, the inventors have identified a need in the art for methods of assessing renal function with increased precision.

### SUMMARY

In one aspect, the invention is directed to an *in vitro* method for determining whether a canine or feline animal is suffering from kidney disease as the result of structural damage or glomerulonephritis, the method comprising measuring β-aminoisobutyric acid (BAIB) in a urine sample or a blood sample from the animal and determining kidney disease based upon the concentration of BAIB in the sample, further comprising comparing the concentration of BAIB in the sample to a reference concentration related to the concentration in BAIB in samples from healthy animals, wherein the measuring comprises contacting the sample with an anti-BAIB antibody and determining binding or the amount of binding between the antibody and BAIB in the sample, further wherein the anti-BAIB antibody is raised against a conjugate comprising BAIB and a carrier protein, BAIB and polylysine, or BAIB and glutaraldehyde
and wherein the structural damage is the result of inflammation, fibrosis, injury, kidney stones, or infiltration by cancer.

In another aspect, the invention relates to an *in vitro* method of diagnosing a kidney disease which is the result of structural damage or glomerulonephritis in a canine or feline animal subject, the method comprising: contacting a urine or blood sample obtained from the subject with an anti-BAIB antibody and determining binding or the amount of binding between the antibody and BAIB in the sample, wherein the antibody is raised against a conjugate comprising BAIB and a carrier protein, BAIB and polylysine, or BAIB and glutaraldehyde; measuring the concentration of BAIB in the sample; comparing the level of BAIB to a reference concentration of BAIB in healthy subjects; and diagnosing a kidney disorder which is a result of structural damage or glomerulonephritis where the value of BAIB in the sample is in excess of the reference concentration and wherein the structural damage is the result of inflammation, fibrosis, injury, or infiltration by cancer or kidney stones.

In the various aspects of the disclosure, the reference concentration can reflect the 95^{th} percentile of the concentration of BAIB in healthy animals. Also, the kidney disease is the result of structural damage. The structural damage may be the result of inflammation, fibrosis, injury, kidney stones (such as oxalate stones) or infiltration by cancers. Alternatively, the kidney disease is glomerulonephritis.

In a further aspect, the invention relates to an *in vitro* method for determining whether a canine or feline animal subject is suffering from kidney disease due to structural damage or glomerulonephritis, the method comprising: measuring the concentration of BAIB and symmetrical dimethyl arginine (SDMA) in a urine or blood sample obtained from the subject; wherein the measuring comprises contacting the sample with an anti-BAIB antibody raised against a conjugate comprising BAIB and a carrier protein, BAIB and polylysine, or BAIB and glutaraldehyde; and determining binding or the amount of binding between the anti-BAIB and BAIB in the sample; and determining kidney disease when the ratio of the concentration of BAIB [BAIB] to concentration of SDMA [SDMA] is greater than 0.15 or when the ratio of [SDMA] to [BAIB] is less than 7 and wherein the structural damage is the result of inflammation, fibrosis, injury, or infiltration by cancer.

Still further, the invention relates to an *in vitro* method of diagnosing a renal disorder which is a loss of kidney function due to structural damage in a canine or feline animal subject, the method comprising: measuring the concentration of one or more of BAIB and SDMA in a urine or blood sample obtained from the subject; wherein the measuring comprises contacting the sample with an anti-BAIB antibody raised against a conjugate of BAIB and a carrier protein, BAIB and polylysine, or BAIB and glutaraldehyde, and determining binding or the amount of binding between the anti-BAIB and BAIB in the sample; comparing the level BAIB and SDMA to reference concentrations of BAIB and SDMA in healthy subjects; diagnosing loss of kidney function when the concentration of SDMA in the sample is in excess of the SDMA reference concentration and diagnosing that the loss of kidney function is a result of structural damage when the concentration of BAIB in the sample is in excess of the BAIB reference concentration and wherein the structural damage is the result of inflammation, fibrosis, injury, kidney stones or infiltration by cancer.

In embodiments, the animal is a feline and the reference concentration is 2.0 µg/dL BAIB, or the animal is a canine and the reference concentration is 1.2 µg/dL BAIB, and/or wherein the reference concentration for SDMA is 14 µg/dL.

Further, the carrier protein is preferably one of KLH and BSA.

The invention also relates to an *in vitro* method of determining the presence or amount of BAIB in a sample, the method comprising contacting an antibody raised against a conjugate of BAIB with the sample and determining binding or the amount of binding between the antibody and BAIB in the sample, further comprising contacting the sample and the antibody with a conjugate comprising BAIB and a detectable label, wherein the sample is a urine sample or a blood sample from a canine or feline animal suffering from kidney disease due to structural damage or glomerulonephritis, further wherein the antibody is raised against a conjugate of BAIB and a carrier protein, BAIB and polylysine, or BAIB and glutaraldehyde and wherein the structural damage is the result of inflammation, fibrosis, injury, or infiltration by cancer.

In embodiments, the blood sample is serum or plasma.

In further embodiments, at least one of the reference concentration of BAIB and the reference concentration of SDMA - if measured - reflect the 95th percentile of the concentration of BAIB and SDMA in samples from healthy animals.

Further, the antibody can comprise a label.

### DESCRIPTION

The disclosure generally relates to the determination of renal function. More particularly, the disclosure relates to methods for diagnosing and determining kidney disease as defined in the appended claims.

In various aspects, the disclosure is directed to the use of β-Aminoisobutyric Acid (BAIB), also known as β-amino isobutyrate, to determine or diagnose kidney disease in a canine or feline animal subject. BAIB is a marker for structural damage of the kidney leading to nephron loss, and therefore a marker for kidney cancer, renal carcinoma, metastatic kidney renal carcinoma, neoplastic infiltration of the kidney, inflammation of the kidney, interstitial hypoplasmocytic nephritis, glomerular inflammation, and kidney fibrosis.

In addition, symmetrical dimethylarginine (SDMA) in blood samples from animals, i. e. cats and dogs, is used along with BAIB for improve prognosis accuracy.

SDMA is the structural isomer of the endogenous nitric oxide synthetase (NOS) inhibitor asymmetrical dimethylarginine (ADMA). Both ADMA and SDMA derive from intranuclear methylation of L-arginine residuals and are released into the cytoplasm after proteolysis. SDMA is produced by protein-arginine methyltransferase 5 (PRMT 5) and PRMT 7. Proteins carrying methylarginines, such as SDMA, ADMA and monomethylarginine, play a role in RNA processing, protein shuttling and signal transduction (Bedford and Richard, Mol. Cell 2005, 18(3):263-72). Free SDMA resulting from the degradation of such methylated proteins is mainly eliminated by renal excretion, whereas ADMA is largely metabolized. ADMA is strongly correlated with risk factors for coronary artery disease (CAD) such as hypertension, hypercholesterolemia, hyperhomocysteinemia, insulin resistance, age, and mean arterial pressure. SDMA is correlated with parameters of renal function, such as glomerular filtration rate (GFR), inulin clearance, and creatinine clearance.

BAIB may be marker for damage or lesions in the kidney, even when kidney function (e.g., as indicated by SDMA) is within the normal reference range.

Before describing further aspects of the disclosure, a number of terms are defined below:
BAIB is β-aminoisobutyric acid (β-amino isobutyrate). The structure of BAIB is:

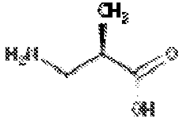

BSA is bovine serum albumin.

Kidney disease, involves the loss of nephron function (filtration efficiency) with or without structural damage. Structural damage may be caused by lesions, inflammation, fibrosis, injury, infiltration, and other sources. While cancer may be a cause of the structural damage, the cancer itself is not generally identified as kidney disease.

Kidney stones refer to stones in any part of renal or urinary anatomy, including nephroliths, nephroliths inside tubule or urether, and stones in the bladder. Kidney stones often lead to structural damage and kidney disease.

CMIA is chemiluminescent magnetic immunoassay.

DIPEA is N,N-diisopropylethylamine.

DMF is dimethyl formamide.

EIA is enzyme immunoassay.

ELISA is enzyme-linked immunosorbent assay.

ESI-MS is electrospray ionization mass spectrometry.

FPIA is fluorescence polarization immunoassay.

GFR is glomerular filtration rate.

HATU is (1H-7-azabenzotriazol-1-yl)-1,1,3,3-tetramethyl uranium hexafluorophosphate methanamininium.

KLH is keyhole limpet hemocyanin.

MEIA is microparticle enzyme immunoassay.

PBS is phosphate buffered saline.

RIA is radioimmunoassay.

SDMA is symmetrical dimethylarginine. The structure of SDMA is:

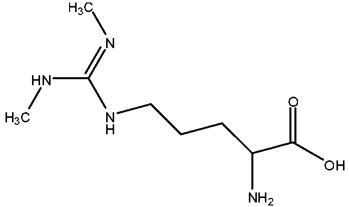

Free SDMA refers to SDMA that is not part of a polypeptide chain. One or more amino acid residues of SDMA can be present in a polypeptide.

TFA is trifluoracetic acid.

The term "analog," as used herein, generally refers to a compound in which one or more individual atoms have been replaced with a different atom(s) or with a different functional group(s). For example, an analog may be a modified form of the analyte which can compete with the analyte for a receptor, the modification providing a means to join the analyte to another moiety, such as a label or solid support. The analyte analog can bind to an antibody in a manner similar to the analyte.

The term "antibody," as used herein, generally refers to a glycoprotein produced by B lymphocyte cells in response to exposure to an antigen and binds specifically to that antigen. The term "antibody" is used in its broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

As used herein, an "anti-BAIB," "anti-BAIB antibody portion," or "anti-BAIB antibody fragment" and/or "anti-BAIB antibody variant" and the like include any protein or peptide containing molecule that comprises at least a portion of an immunoglobulin molecule, such as, but not limited to, one complementarity determining region (CDR) of a heavy chain or light chain constant region, a framework region, or any portion thereof, that specifically finds to BAIB.

As used herein, an "anti-SDMA," "anti-SDMA antibody portion," or "anti-SDMA antibody fragment" and/or "anti-SDMA antibody variant" and the like include any protein or peptide containing molecule that comprises at least a portion of an immunoglobulin molecule, such as, but not limited to, one complementarity determining region (CDR) of a heavy chain or light chain constant region, a framework region, or any portion thereof, that specifically binds to SDMA.

The term "antibody fragment," as used herein, refers to a portion of a full length antibody, generally the antigen binding or variable domain thereof. Specifically, for example, antibody fragments may include Fab, Fab', F(ab')₂, and Fv fragments; diabodies; linear antibodies; single-chain antibody molecules; and multispecific antibodies from antibody fragments.

The term "antigen," as used herein, generally refers to a substance that is capable, under appropriate conditions, of reacting with an antibody specific for the antigen.

The term "analyte," as used herein, generally refers to the substance, or set of substances in a sample that are detected and/or measured.

The term "animal," as used herein, generally refers to a non-human animal which is a canine or a feline.

The term "sample," as used herein, generally refers to urine or any blood-derived fluid sample, including but not limited to whole blood, plasma, and serum. To provide serum for use in the methods of the disclosure, one or more serum samples are obtained from the animal subject. The serum samples can be, for example, obtained from the animal subject as blood samples, then separated to provide serum. In certain embodiments, the serum can be measured without separation from blood. As the person of skill in the art will appreciate, a single obtained sample can be divided or otherwise used to do numerous concentration measurements. Alternatively, a plurality of samples can be obtained from the animal subject, with (at least) one sample being measured for each analyte of interest, or for numerous analytes sequentially, in parallel, or simultaneously. In certain such cases, the samples are obtained from the animal at about the same time (e.g., within 60 minutes, within 30 minutes, or even within 10 minutes of one another).

The term "cross-reactivity," as used herein, generally refers to the ability of an individual antigen binding site of an antibody to react with more than one antigenic determinant or the ability of a population of antibody molecules to react with more than one antigen. In general, cross reactions arise because (i) the cross reacting antigen shares an epitope in common with the immunizing antigen or (ii) it has an epitope which is structurally similar to one on the immunizing antigen (multi specificity).

The term "immunoassay," as used herein, generally refers to a test that employs antibody and antigen complexes to generate a measurable response. An "antibody:antigen complex" may be used interchangeably with the term "immuno-complex." Immunoassays, in general, include noncompetitive immunoassays, competitive immunoassays, homogeneous immunoassays, and heterogeneous immunoassays. In "competitive immunoassays," unlabeled analyte (or antigen) in the test sample is measured by its ability to compete with labeled antigen in the immunoassay. The unlabeled antigen blocks the ability of the labeled antigen to bind because the binding site on the antibody is already occupied. In "competitive immunoassays," the amount of antigen present in the test sample is inversely related to the amount of signal generated from the label. Immunoassays that require separation of bound antibody:antigen complexes are generally referred to as "heterogeneous immunoassays," and immunoassays that do not require separation of antibody: antigen complexes are generally referred to as "homogeneous immunoassays." One of skill in the art would readily understand the various immunoassay formats.

"Contacting" as used here is used in its broadest aspect to refer to combining reagents in any order unless otherwise specified herein.

The term "immune complexes," as used herein, generally refers to the complexes formed by the binding of antigen and antibody molecules, with or without complement fixation. When one of either the antibody or antigen is labeled, the label is associated with the immune complex as a result of the binding between the antigen and antibody. Therefore, when the antibody is labeled, the label becomes associated with the antigen as a result of the binding. Similarly, when the antigen is labeled (*e.g.,* an analyte analog having a label), the label becomes associated with the antibody as a result of the binding between the antigen and the antibody.

The term "label," as used herein, refers to a detectable compound, composition, or solid support, which can be conjugated directly or indirectly (e.g., via covalent or non-covalent means, alone or encapsulated) to an antibody, BAIB analog, SDMA analog, or antigen of the disclosure. The label may be detectable by itself (e.g., radioisotope labels, chemiluminescent dye, electrochemical labels, metal chelates, latex particles, or fluorescent labels) or, in the case of an enzymatic label, may catalyze chemical alteration of a substrate compound or composition which is detectable (*e.g.*, enzymes such as horseradish peroxidase, alkaline phosphatase, and the like). The label employed in the current disclosure could be, but is not limited to: alkaline phosphatase; glucose-6-phosphate dehydrogenase ("G6PDH"); horse radish peroxidase (HRP); chemiluminescers such as isoluminol, fluorescers such as fluorescein and rhodamine compounds; ribozymes; and dyes. The label may also be a specific binding molecule which itself may be detectable (e.g., biotin, avidin, streptavidin, digioxigenin, maltose, oligohistidine, 2, 4-dinitrobenzene, phenylarsenate, ssDNA, dsDNA, and the like). The label may be bound to another molecule or solid support and that is chosen for specific characteristics that allow detection of the labeled molecule. The utilization of a label produces a signal that may be detected by means such as detection of electromagnetic radiation or direct visualization, and that can optionally be measured.

The term "monoclonal antibody," as used herein generally refers to an antibody obtained from a population of substantially homogeneous antibodies, *i.e.,* the individual antibodies comprising the population are identical. Monoclonal antibodies are highly specific, being directed against a single antigenic site. In contrast to polyclonal antibody preparations, which typically include different antibodies directed against different epitopes, each monoclonal antibody is directed against a single epitope on the antigen. The modifier "monoclonal" merely refers to the character of the antibody and is not to be construed as requiring production of the antibody by any particular method. Specifically, for example, monoclonal antibodies may be made by hybridoma methodologies, or may be made by recombinant DNA methods, or may be isolated from phage antibody libraries using known techniques.

The term "polypeptide," as used herein, generally refers to a molecule having a sequence of amino acids linked by peptide bonds. This term includes proteins, fusion proteins, oligopeptides, cyclic peptides, and polypeptide derivatives. Antibodies and antibody derivatives are discussed above in a separate section, but antibodies and antibody derivatives are, for purposes of the disclosure, treated as a subclass of the polypeptides and polypeptide derivatives.

The term "solid support," as used herein, refers to a non-aqueous matrix to which the antibody or SDMA analog of the present disclosure can adhere. Examples of solid support include supports formed partially or entirely of glass (e.g., controlled pore glass), synthetic and natural polymers, polysaccharides (e.g., agarose), polyacrylamides, polystyrene, polyvinyl alcohols and silicones, magnetic particles, latex particles, chromatographic strips, microtiter polystyrene plates, or any other substances that will allow bound antigens and/or antibodies to be washed or separated from unbound materials. In certain embodiments, depending on the application, the solid support can be the well of an assay plate or can be a purification column (*e.g.,* an affinity chromatography column).

"Receptor" refers to any compound or composition capable of recognizing a particular spatial and polar organization of a molecule, e.g., epitopic or determinant site. Illustrative receptors include antibodies, Fab fragments, and the like.

"Binding specificity" or "specific binding" refers to the substantial recognition of a first molecule for a second molecule, for example a polypeptide and a polyclonal or monoclonal antibody, or an antibody fragment (e.g. a Fv, single chain Fv, Fab', or F(ab')2 fragment) specific for the polypeptide. For example, "specificity," as used herein, generally refers to the ability of an individual antibody combining site to react with only one antigenic determinant or the ability of a population of antibody molecules to react with only one antigen. In general, there is a high degree of specificity in antigen-antibody reactions. Antibodies can distinguish differences in (i) the primary structure of an antigen, (ii) isomeric forms of an antigen, and (iii) secondary and tertiary structure of an antigen. Antibody-antigen reactions that exhibit high specificity exhibit low cross reactivity.

"Substantial binding" or "substantially bind" refers to an amount of specific binding or recognizing between molecules in an assay mixture under particular assay conditions. In its broadest aspect, substantial binding relates to the difference between a first molecule's incapability of binding or recognizing a second molecule, and the first molecules capability of binding or recognizing a third molecule, such that the difference is sufficient to allow a meaningful assay to be conducted distinguishing specific binding under a particular set of assay conditions, which includes the relative concentrations of the molecules, and the time and temperature of an incubation. In another aspect, one molecule is substantially incapable of binding or recognizing another molecule in a cross-reactivity sense where the first molecule exhibits a reactivity for a second molecule that is less than 25%, less than 10%, less than 5% or less than 1% of the reactivity exhibited toward a third molecule under a particular set of assay conditions. Specific binding can be tested using a number of widely known methods, e.g., an immunohistochemical assay, an enzyme-linked immunosorbent assay (ELISA), a radioimmunoassay (RIA), or a western blot assay.

The term "salt," as used herein, means a salt formed between an acid and a basic functional group of a compound. Illustrative salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts. The term "salt" also refers to a salt formed between a compound having an acidic functional group, such as a carboxylic acid functional group, and an inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxysubstituted mono-, di-, or trialkylamines; dicyclohexylamine; tributyl amine; pyridine; N-methyl, N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N,N,-dimethyl-N-(2-hydroxyethyl)amine, or tri-(2-hydroxyethyl)amine; N-methyl-D-glucamine; and amino acids such as arginine, lysine, and the like.

Continuing with the invention, in one aspect, the invention relates to an *in vitro* method for determining whether a canine or feline animal is suffering from kidney disease as the result of structural damage or glomerulonephritis, the method comprising measuring BAIB in a urine sample or a blood sample from the animal and determining kidney disease based upon the concentration of BAIB in the sample, further comprising comparing the concentration of BAIB in the sample to a reference concentration related to the concentration in BAIB in samples from healthy animals, wherein the measuring comprises contacting the sample with an anti-BAIB antibody and determining binding or the amount of binding between the antibody and BAIB in the sample, further wherein the anti-BAIB antibody is raised against a conjugate comprising BAIB and a carrier protein, BAIB and polylysine, or BAIB and glutaraldehyde; and wherein the structural damage is the result of inflammation, fibrosis, injury, kidney stones or infiltration by cancer. one aspect, an upper reference limit represents the 95th percentile from a population of the healthy feline subjects. Subject having blood or urine BAIB concentration [BAIB] above the reference limit may be considered as suffering from a renal disorder, such as kidney structural damage. For feline subjects, the 95^{th} percentile reference limit may be about 2.0 µg/dL BAIB for a population of felines. Ranges for the 95^{th} percentile can include, for example about 1.5-2.5 µg/dL, in particular about 1.5, 1.6, 1.7, 1.8, 1.9, 2.0, 2.1, 2.2, 2.3, 2.4 and 2.5 µg/dL. For canines, the 95^{th} percentile is about 1.0-2.0, in particular 1 about .0, 1.1, 1.2, 1.3, 1.4, 1.5, 1.6, 1.7, 1.8, 1.9, and 2.0 µg/dL.

Another aspect of the disclosure includes using a combination of BAIB and SDMA to determine renal disorders. In this aspect, reference ranges for both BAIB and SDMA can be known or obtained from a population of healthy subjects. A value for BAIB concentration and SDMA concentration above the reference value may be indicative of renal disorder. An appropriate upper reference limit representing the 95^{th} percentile in healthy subjects for SDMA may be, for example, 14 µg/dL. *See* WO2015/035115. A [BAIB]/[SDMA] ratio of greater than 0.15, or an [SMDASDMA]/[BAIB] ratio of less than 7, is indicative of renal disorder. Elevated BAIB concentrations in the presence of normal SDMA concentrations can indicate structural damage even with normal kidney function. Decreased BAIB concentrations in the presence of elevated SDMA concentration can be indicative of loss of nephron function in the absence of structural damage.

Also described herein is a method of diagnosing kidney stones. The method includes measuring the concentration of BAIB in a sample from a subject and determining if the subject has a BAIB concentration above a reference limit. Elevated BAIB levels above the reference limit are indicative of structural damage, which may be caused by kidney stones. Further analysis by ultrasound, CT-scan, or x-ray of the kidney can be confirmatory in the absence of other symptoms of kidney stones.

The methods of the invention can be applied to determine the concentration of BAIB in two or more samples obtained from the respective subject, over the course of minutes, hours, days, week, months or years. Kidney disease diagnosis or progression can be determined based upon the concentration of BAIB in the samples. When the concentration of BAIB in the samples is increasing over the series of samples, it can be determined that the kidney disease, such as kidney structural damage, is worsening. Mortality or early death may also be predicted based upon the increasing concentration of BAIB in the series of samples.

The disclosure is also directed to a computing device for performing the calculation or determining ratios as described herein or for diagnosing kidney disease or dysfunction. The computing device includes memory storage for software instructions, which when executed, calculate a value from an equation that can be used to lead to a determination of a disease state.

In certain embodiments, the concentration of free SDMA is determined using the immunological methods, devices and kits described in U.S. Patent No. 8,481,690, WO2015/035155, and US Provisional Patent Application 62/118,832 filed February 20, 2015. The method may include controls, calibrators or standards comprising one or more SDMA analogs. In particular, the method may be accomplished using immunoassay techniques well known to those of skill in the art, including, but not limited to, using microplates and lateral flow devices. Animal subjects from which samples are obtained for detecting SDMA, include non-human canine and feline animal subjects

Samples may be analyzed using a modified assay based upon the EMIT^{®}(Enzyme Multiplied Immunoassay Technique) homogeneous immunoassay system. In a traditional EMIT^{®} assay, a sample containing the analyte is contacted with an anti-analyte antibody, a conjugate of the analyte and an enzyme, and a substrate that produces a signal when in contact with the enzyme. Binding of the antibody to the conjugate inhibits or reduces enzyme activity. When analyte is present in the sample, the sample analyte competes with the conjugated analyte for binding to the antibody, which results in the generation of more signal from the enzyme/substrate. When no analyte is present, more binding can occur between antibody and conjugate to limit or prevent signal generation. Therefore, more signal is generated when more analyte is present. Kinetic assays can use the rate of signal generation as an indicator of the presence or amount of analyte in a sample

In one aspect, signal is measured as absorbance at a wavelength specific for an enzyme/substrate system as is well known in the art. For instance, measurement of absorbance at 340 nm for a G6PDH/NAD enzyme/substrate system will provide a value for the relative amount of conversion of NAD to NADH in the presence of G6PDH, which can be used to provide a reaction rate reflecting the conversion of the substrate by the enzyme.

Reaction rate can be determined by measuring signal (e.g. absorbance) at a plurality of time points during the enzyme mediated reaction. Determination of the time and interval of signal measurement are within the skill in the art taking into consideration the concentration of the reagents and the temperature of the assay. For instances, a rate can be determined by measuring absorbance beginning about 2-10 minutes after the combination of sample (or calibrator) and all reagents at room temperature and measured every 5-60 seconds for an additional 1-15 minutes. Reaction rate can be expressed as the change in absorbance over time. For example, absorbance can be measured starting at about 1, 2, 3, 4, 5, 6, 7, 8, 9, or 10 minutes after combining the sample (or calibrator) and all the reagents. Absorbance is typically measured at intervals of 5, 10, 15, 20, 25, 30, 35, 40, 45, 50, 55 or 60 seconds for about 2, 3, 4, 5, 6, 7, 8, 9, 10, 11, 12, 13, 14 or 15 minutes. Each of these times can be extended or shortened, depending on reaction conditions, analyte, and reagents.

According to one embodiment of the disclosure, the analyte is BAIB or SDMA, and the enzyme-conjugate system is G6PDH/NAD. *See* U.S. provisional patent application 62/118,832 filed February 20, 2015 and U.S. patent application 15/048,209 filed February 19, 2016. . In this embodiment, an analog of BAIB or SDMA is conjugated to G6PDH and used as the conjugate in the assays in order to determine the presence or amount of BAIB or SDMA in serum or plasma samples from animals such as humans, cats and dogs. In one aspect of this embodiment, calibrators are prepared by combining known amounts of BAIB or SDMA with in a calibrator matrix (*e.g*., stripped serum).

Also, a solid phase assay format is a commonly used binding assay technique. There are a number of assay devices and procedures wherein the presence of an analyte (*e.g*., BAIB or SDMA) is indicated by the analyte's binding to a conjugate and/or an immobilized complementary binding member. In one particular aspect, the immobilized binding member (*e.g.*, anti-BAIB or anti-SDMA antibody) is bound, or becomes bound during the assay, to a solid phase such as a reaction well, dipstick, test strip, flow-through pad, paper, fiber matrix or other suitable solid phase material. The binding reaction between BAIB or SDMA, in the sample and immobilized antibody is determined by adding to the sample an amount of an analog of BAIB or SDMA, which includes BAIB or SDMA conjugated to a label. After contacting the mixture of the sample and the BAIB or SDMA analog to the solid phase, the mixture and solid phase are incubated to allow for binding between the immobilized antibody, the BAIB or SDMA, and the BAIB or SDMA analog. Following the incubation, unbound reactants are removed from the solid phase. The amount of the label that becomes associated with the antibody through binding of the antibody to the analog is measured. The amount of the label associated with the antibody is inversely proportional to the amount of BAIB or SDMA in the sample. In certain embodiments, BAIB and SDMA are differentially labeled and measured simultaneously according to the disclosure above. In other embodiments, BAIB and SDMA are measured alone, sequentially, or in parallel.

Immobilization of one or more antibodies to BAIB or SDMA onto a device or solid support is performed so that the antibodies will not be washed away by the sample, diluent and/or wash procedures. One or more antibodies can be attached to a surface by physical adsorption (*i.e.,* without the use of chemical linkers) or by chemical binding (*i.e.,* with the use of chemical linkers). Chemical binding can generate stronger attachment of antibodies on a surface and provide defined orientation and conformation of the surface-bound molecules.

In another embodiment, BAIB or SDMA antibodies raised in a particular species are bound to a solid support by interaction with an anti-species antibody that is bound to the support. In one particular aspect, anti-BAIB or anti-SDMA antibodies are raised in rabbits, and the support has bound thereto anti-rabbit antibody that recognizes the anti-BAIB or anti-SDMA antibody raised in rabbits. In this aspect, the antibody may be in the form of anti-serum obtained from the species. The anti-BAIB or anti-SDMA antibodies can either be applied to the solid phase having the anti-species antibody prior to adding the sample to the solid phase, or the anti-BAIB or anti-SDMA antibodies can be mixed with the sample prior to adding the sample to the solid phase. In either case, the anti-BAIB or anti-SDMA antibodies become bound to the solid phase through binding to the anti-species antibody on the solid phase.

In another embodiment, one or more labeled antibodies can be mixed with a test sample prior to application of the mixture to a solid support. In this case, a BAIB or SDMA analog can be attached to the solid support so that the analog will not be washed away by the sample, diluent and/or wash procedures. Labeled antibodies in the sample bind to BAIB or SDMA in the sample and are, therefore, not available for binding with the BAIB or SDMA analog on the solid support. After application of the mixture to the solid support, and an appropriate incubation, the mixture is washed from the solid support. Antibodies that have not bound to sample BAIB or SDMA will become bound to the BAIB or SDMA analog on the solid support. The presence or amount of BAIB or SDMA in the sample is inversely proportional to the amount of antibody that has become bound to the BAIB or SDMA analog. The signal associated with the label on the antibody can be measured by the appropriate method.

Detection of the antibody:antigen complexes may be achieved through a variety of techniques well known in the art, such as, for example, turbidimetry, enzymatic labeling, radiolabeling, luminescence, or fluorescence. Immunoassay methodologies are known by those of ordinary skill in the art and are appreciated to include, but not limited to, radioimmunoassay (RIA), enzyme immunoassays (EIA), fluorescence polarization immunoassays (FPIA), microparticle enzyme immunoassays (MEIA), enzyme multiplied immunoassay technology (EMIT) assays, immuno turbidometric or agglutination assays, colloidal gold based immunoassays including lateral flow devices and chemiluminescent magnetic immunoassays (CMIA). In RIA, an antibody or antigen is labeled with radioactivity and used in a competitive or noncompetitive format. In EIA, an antibody or antigen is labeled with an enzyme that converts a substrate to a product with a resulting signal that is measured, such as a change in color. In FPIA, an antigen is labeled with fluorescent label and competes with unlabeled antigen from the specimen. The amount of analyte measured is inversely proportional to the amount of signal measured. In MEIA, a solid phase microparticle is coated with antibodies against an antigen of interest and is used to capture the analyte. The antibody for detection is labeled with an enzyme as in the EIA method. The concentration of analyte measured is proportional to the amount of signal measured. In CMIA, a chemiluminescent label is conjugated to the antibody or antigen, and produces light when combined with its substrate. CMIA can be configured in a competitive or noncompetitive format, and yields results that are inversely or directly proportional to the amount of analyte present, respectively.

The use of reagent-impregnated test strips in specific binding assays is also well-known. In such procedures, a test sample is applied to one portion of the test strip and is allowed to migrate or wick through the strip material. Thus, the analyte to be detected or measured passes through or along the material, possibly with the aid of an eluting solvent which can be the test sample itself or a separately added solution. The analyte migrates into a capture or detection zone on the test strip, wherein a complementary binding member to the analyte is immobilized. The extent to which the analyte becomes bound in the detection zone can be determined with the aid of the conjugate which can also be incorporated in the test strip or which can be applied separately. In one embodiment, an antibody specific for BAIB or SDMA is immobilized on a solid support at a distinct location. Following addition of the sample, detection of BAIB- or SDMA -antibody complexes on the solid support can be by any means known in the art. For example, U.S. Patent No. 5,726,010, describes an example of a lateral flow device, the SNAP^{®} immunoassay device (IDEXX Laboratories).

Other detection technologies employ magnetic particles or microbeads, for example, superparamagnetic iron oxide impregnated polymer beads. These beads are associated with, for example, a specific binding partner for the analyte. The beads bind with the target analytes in the sample being tested and are then typically isolated or separated out of solution magnetically. Once isolation has occurred, other testing may be conducted, including observing particular images or labels, whether directly optically or by means of a camera.

In a further embodiments, BAIB or SDMA analogs, particularly thiol-containing, hydroxyl-containing, amino containing, and carboxylate containing BAIB or SDMA analogs, enable the BAIB or SDMA to be linked to another molecule (conjugation target), such as an activated protein, to form an BAIB or SDMA conjugate. The BAIB or SDMA analogs described herein enable BAIB or SDMA to be linked to a conjugation target such as a protein, polypeptide, detectable label, solid support, and the like to provide the BAIB or SDMA conjugate. The BAIB or SDMA conjugates described herein can be used to produce antibodies for use in immunoassays specific for BAIB or SDMA. The BAIB or SDMA analogs can also be conjugated to a label for use in immunoassays specific for BAIB or SDMA.

To raise an anti-BAIB antibody, BAIB is conjugated to a carrier protein to form a "hapten-carrier" immunogen that can be used to stimulate an immune response to an epitope that includes BAIB. Exemplary immunogenic proteins include, but are not limited to, BSA, KLH, and ovalbumin. Protocols for conjugating haptens to immunogenic proteins are known in the art (*see, e.g.,* Antibodies: A Laboratory Manual, E. Harlow and D. Lane, eds., Cold Spring Harbor Laboratory (Cold Spring Harbor, NY, 1988) pp. 78-87).

Suitable SDMA analogs are described, for example, in U.S. Patent 8,481,690 and WO2015/035155 .

The BAIB analogs include, for example, BAIB-thiol (amino end and carboxyl end) and BAIB-FMOC (Fluorenylmethyloxycarbonyl), shown here:

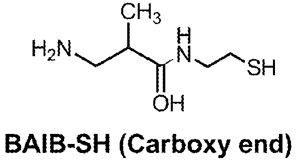

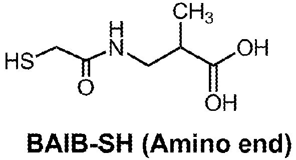

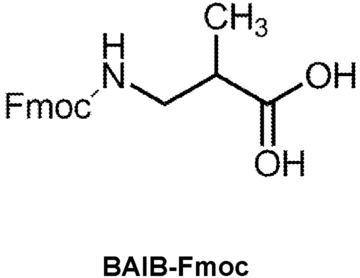

These analogs can be used to prepare conjugates of BAIB including, for example, the following: a glutaraldehyde-BAIB conjugate, a poly-lysine-BAIB conjugate, a BAIB(amine end)-BSA conjugate, a BAIB (carboxy end)-BSA conjugate; a BAIB(amine end)-KLH conjugate, a BAIB(Carboxy end)-KLH conjugate, a BAIB-G6PDH conjugate, and BAIB conjugated to a particle. Structures for several of these conjugates are shown here.

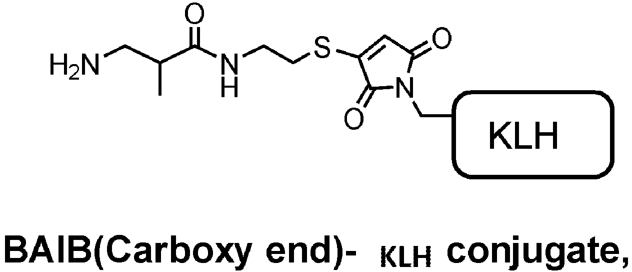

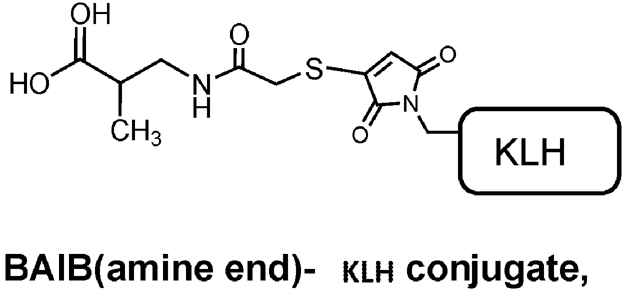

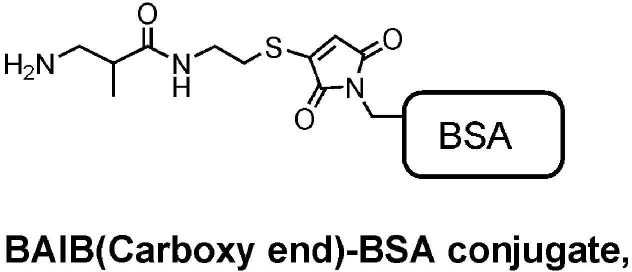

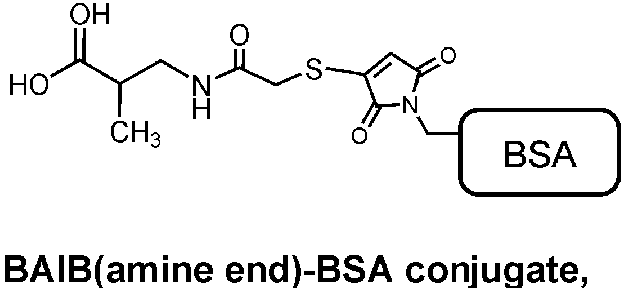

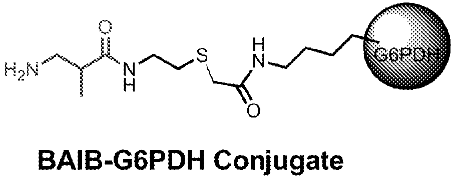

In an alternate embodiment, the BAIB and/or SDMA analogs are linked to a detectable label. The label may be detectable by itself *(e.g*., radioisotope labels, chemiluminescent dye, electrochemical labels, metal chelates, latex particles, or fluorescent labels) or, in the case of an enzymatic label, may catalyze chemical alteration of a substrate compound or composition which is detectable (*e.g.*, enzymes such as horseradish peroxidase, alkaline phosphatase, and the like). The label may be a specific binding molecule which itself may be detectable (*e.g.*, biotin, avidin, streptavidin, digioxigenin, maltose, oligohistidine, 2, 4-dinitrobenzene, phenylarsenate, ssDNA, dsDNA, etc.). The SDMA and/or BAIB can be linked to a detectable label using methods well known to those skilled in the art.

A conjugate of the analogs of SDMA and BAIB and KLH or BSA are used as an immunogen to generate antibodies that substantially bind BAIB and SDMA (*i.e.,* anti-BAIB and SDMA antibodies). Anti-SDMA antibodies and anti-BAIB antibodies useful in the methods of the disclosure are characterized by a high affinity binding to BAIB and SDMA. Accordingly, described herein are isolated, recombinant, synthetic, and/or *in vivo*produced anti-BAIB antibodies and anti-SDMA antibodies, as well as methods of making and using such antibodies. The antibodies described herein are useful, for example, as reagents in assays for determination of SDMA and BAIB in canine or feline animal subject samples. In one embodiment, the generated antibodies are able to detect BAIB and free SDMA (*i.e.,* SDMA not part of a polypeptide chain).

The methods for making the antibodies include using one or more BAIB and SDMA conjugates as immunogens to stimulate an immune response. The methods include administering one or more BAIB and SDMA conjugates to an animal using a suitable immunization protocol, and separating an appropriate antibody from a body fluid(s) of the animal. Alternatively, the conjugates may be used in phage display methods to select phage displaying on their surface an appropriate antibody, followed by separation of nucleic acid sequences encoding at least a variable domain region of an appropriate antibody. Phage display methods are well known to those of ordinary skill in the art. (*See, for example,* Antibody Phage Display; Methods in Molecular Biology, Vol. 178, O'Brien, Philippa M.; Aitken, Robert (Eds.) 2002). Monoclonal antibodies to can be prepared by methods generally known in the art.

The BAIB and SDMA analogs described herein may be linked to a label to provide a detectable conjugate for use in receptor binding assays, such as immunoassays for BAIB and SDMA. Similarly, the anti-BAIB and anti-SDMA antibodies can be linked to a label to provide detectable anti-SDMA antibodies and anti-BAIB antibodies for use in receptor binding assays, such as immunoassays. The analogs and antibodies can be linked to a label using methods well known to those skilled in the art. *E.g.,* Immunochemical Protocols; Methods in Molecular Biology, Vol. 295, edited by R. Burns (2005)). The detectable conjugates or detectable anti-SDMA antibodies may be used in various homogenous and/or competitive assay formats to generate a signal that is related to the presence or amount of BAIB and/or SDMA in a test sample.

In a specific embodiment, the immunoassay methodologies are competitive immunoassays for detection of anti-BAIB and anti-SDMA antibodies. The competitive immunoassay may be carried out in the following illustrative manner. A sample, from an animal's body fluid is contacted with a BAIB analog or an SDMA analog conjugated to a solid support and with an anti-BAIB and anti-SDMA antibody conjugated to a detectable label. The antibodies of interest, present in the sample, compete with the anti-BAIB and anti-SDMA antibodies conjugated to a detectable label for binding with the analogs conjugated to a solid support. The amount of the label associated with the solid support can be determined after separating unbound antibodies and the solid support. In an alternative embodiment, the competitive immunoassay is carried out in the following illustrative manner. A sample, from an animal's body fluid, containing BAIB and SDMA, is contacted with a BAIB analog or an SDMA analog linked to a detectable label and then with an anti-BAIB or anti-SDMA antibody conjugated to a solid support. The SDMA or BAIB in the sample compete with the SDMA or BAIB on the solid support for binding with the BAIB or SDMA conjugate linked to a labeled antibody. In either case, the signal obtained is inversely related to the amount of BAIB or SDMA present in the sample.

As described herein, the concentration of creatinine in serum can be measured in a variety of ways, as is known by the person of skill in the art. For example, a Catalyst Dx^{™} Chemistry Analyzer or a VetTest^{®} Chemistry Analyzer can be used with dry-slides adapted to test for creatinine, for example, those commercially available from IDEXX Laboratories. Other analyzers and slides, such as the VITROS^{®} 950 analyzer and VITROS^{®} CREA slides available from Ortho Clinical Diagnostics, and the COBAS^{®} analyzer and related kits from Roche Diagnostics, can also be used. Enzymatic wet assays can also be used. For example, the person of skill in the art can use an enzymatic wet chemistry method on an Integra 800 analyzer. One particular assay is based on a creatininase/creatinase/sarcosine oxidase system with detection at 552 nm and absorbance blanking at 659 nm. The person of skill in the art can also use colorimetric methods, for example, those based on picrate such as the Jaffe assay. Other methods known to the person of skill in the art, such as those described in U.S. Patent Publication no. 2005/0266574 and U.S. Patent no. 4,818,703, can also be used to measure creatinine concentration. In certain aspects, the measurement of creatinine concentration is performed using isotope dilution mass spectrometry.

### EXAMPLES

### Example 1: Liquid Chromatography Mass Spectrometry (LC-MS) assay for BAIB serum levels (comparative example, not part of the invention)

A Liquid Chromatography Mass Spectrometry (LC-MS) assay was optimized for measuring BAIB serum levels.

Canine stripped serum was prepared as follows: untreated commercial canine serum (500 mL) was loaded to a two foot SNAKESKIN^{™} Dialysis tube (3.5 K MWCO, 35 mm Dry I.D.)(Thermo Scientific) and dialyzed against PBS buffer (20 L) with 20 g carbon powder at 4 °C for at least six hours. The process was repeated three times by changing buffer and carbon. The BAIB concentration in the serum was measured by LC-MS before and after dialysis. In the serum before dialysis, BAIB concentration was 2-3 µg/dL. After dialysis, BAIB concentration was below the detection limit. The charcoaled stripped canine serum was stored at -80 °C for use.

An LC-MS standard curve was generated according to the following procedure.

An internal standard was prepared dissolving 50 µg/dL D3-BAIB (BAIB labeled with 3 deuterium atoms)(CDN ISOTOPES Product Number D-7229) in water.

Assay standards were prepared by first preparing a solution of 1 mg/mL BAIB in water. 8 µL of this solution was transferred into 7992 µL of stripped canine serum and serially diluted into stripped serum (prepared above) to generate the dilution series in Table 1.

**Table 1**

| **ID** | **[BAIB] µg/dL** |
|---|---|
| STD 1 | 0.78 |
| STD 2 | 1.56 |
| STD 3 | 3.13 |
| STD 4 | 6.25 |
| STD 5 | 12.5 |
| STD 6 | 25 |
| STD 7 | 50 |
| STD 8 | 100 |

Samples were prepared for LC-MS according to the following:
1. 50 µL of test samples or reference standards were transferred into vials.
2. 50 µL of the internal standard solution was added to the vial and the solution mixed thoroughly.
3. 300 µL of pure acetonitrile was added to the vial and the solution mixed thoroughly.
4. Vials were centrifuge at 3000 xg for 20 minutes, the supernatant was decanted, filtered (0.2 µm), and subjected to LC-MS under conditions described below.

LC-MS was run under the following conditions:

| | |
|---|---|
| Mobile Phase A: | Water, 0.1% formic acid, 0.5mM Perfloroheptanoic acid (Sigma 342041-5G) |
| Mobile Phase B: | Acetonitrile 0.1% formic acid |
| Column: | Acquity CSH C18 1.7 µm, 2.1x30mm (Waters 186005295) |
| Scan Type: | MRM |
| Scan Mode: | Positive |
| Ion source: | Turbo Spray |

| Analyte | Q1 Mass (Da) | Q3 Mass (Da) | Dwell (msec) | DP | CEC | XP |
|---|---|---|---|---|---|---|
| BAIB | 104.1 | 85.900 | 15 | 41 | 19 | 12 |
| D3-BAIB | 107.1 | 88.9 | 15 | 41 | 19 | 12 |

| | |
|---|---|
| INJ vol: | 2µL |
| Flow: | 1mL/min |
| Column temp: | 20 deg. C. |
| Cooler temp: | 15 deg. C. |
| Initial %B: | 0% |

### Time program

| Time (min) | Module | Events | Parameter |
|---|---|---|---|
| 1.00 | Pumps | Pump B Conc. | 5 |
| 2.20 | Pumps | Pump B Conc. | 100 |
| 2.50 | Pumps | Pump B Conc. | 100 |
| 2.60 | Pumps | Pump B Conc. | 0 |
| 4.00 | | System controller | Stop |

Start at 0% B at time 0 min. Ramp up gradient to 5% B in 1 min. Ramp up the gradient to 100% B for 1.2 min (2.20 min mark). Stay at 100% B for 0.3 min (2.5 min mark) then back to the initial conditions (0% B).

### Example 2: Measurement of BAIB in Healthy and Diseased Felines (comparative example, not part of the invention)

BAIB reference levels in normal feline subjects were determined in 58 cats of both sexes, and from various breeds. Serum samples were collected, subjected to LC-MS as described above, and individual test samples were compared to standards (measured above) to determine BAIB levels. The upper reference limit based on the 95th percentile for this population was 2.0 µg/dL.

Serum BAIB concentration [BAIB] in felines suffering from kidney disease was also measured in ten felines by LC-MS. In addition, SDMA concentration [SDMA] was measured by LC-MS.

The levels of BAIB and SDMA are show in Table 2.

**Table 2**

| **Sample Date*** | **SDMA µg/dL** | **BAIB µg/dL** | **Discordance** | **SDMA: BAIB** | **BAIB: SDMA** |
|---|---|---|---|---|---|
| Week 0 | 14 | 0 | No | | 0 |
| Week 63 | 10 | 1.2 | No | 8 | 0 |
| Week 105 | 14 | 0 | No | | 0 |
| Week 115 | 17 | 0 | No | | 0 |
| Week 125 | 13 | 2.3 | No | 6 | 0 |
| Week 131 | 13 | 0 | No | | 0 |
| Week 139 | 13 | 1.35 | No | 10 | 0 |

| **Feline -2** | | | | | |
|---|---|---|---|---|---|
| Week 0 | 12 | 0.404 | No | 30 | 0 |
| Week 57 | 14 | 0.887 | No | 16 | 0 |
| Week 74 | 19 | 0 | No | | 0 |
| Week 84 | 18 | 0 | No | | 0 |
| Week 90 | 15 | 0 | No | | 0 |
| Week 98 | 17 | 0 | No | | 0 |

| **Feline-3** | | | | | |
|---|---|---|---|---|---|
| Week 0 | 17 | 3.12 | No | 5 | 0 |
| Week 76 | 18 | 2.39 | No | 8 | 0 |
| Week 97 | 16 | 0 | No | | 0 |
| Week 105 | 18 | 0 | No | | 0 |
| Week 117 | 15 | 0 | No | | 0 |
| Week 123 | 14 | 0 | No | | 0 |
| Week 131 | 14 | 0 | No | | 0 |

| **Feline-4** | | | | | |
|---|---|---|---|---|---|
| Week 0 | 20 | 3.67 | No | 5 | 0 |
| Week 71 | 23 | 0 | No | | 0 |
| Week 74 | 21 | 0 | No | | 0 |
| Week 84 | 18 | 1.3 | No | 14 | 0 |
| Week 90 | 20 | 0 | No | | 0 |
| Week 98 | 19 | 0 | No | | 0 |

| **Feline-5** | | | | | |
|---|---|---|---|---|---|
| Week 0 | 9 | 0.152 | No | 59 | 0 |
| Week 45 | 16 | 0 | No | | 0 |
| Week 98 | 28 | 0 | No | | 0 |
| Week 108 | 25 | 0 | No | | 0 |
| Week 114 | 28 | 0 | No | | 0 |
| Week 122 | 29 | 0 | No | | 0 |

| **Feline-6** | | | | | |
|---|---|---|---|---|---|
| Week 0 | 12 | 16.3 | No | 1 | 1 |
| Week 53 | 13 | 4.46 | No | 3 | 0 |
| Week 111 | 16 | 9.99 | No | 2 | 1 |
| Week 121 | 17 | 10.9 | No | 2 | 1 |
| Week 131 | 17 | 13.2 | Yes | 1 | 1 |
| Week 137 | 15 | 13.2 | Yes | 1 | 1 |
| Week 145 | 25 | 28.3 | Yes | 1 | 1 |

| **Feline-7** | | | | | |
|---|---|---|---|---|---|
| Week 0 | 26 | 26.2 | Yes | 1 | 1 |
| Week 52 | 38 | 42.5 | Yes | 1 | 1 |
| Week 85 | 43 | 51.5 | Yes | 1 | 1 |
| Week 95 | 42 | 68.5 | Yes | 1 | 2 |
| Week 101 | 41 | 65.6 | Yes | 1 | 2 |
| Week 109 | 44 | 57.7 | Yes | 1 | 1 |

| **Feline-8** | | | | | |
|---|---|---|---|---|---|
| Week 0 | 9 | 0 | No | | 0 |
| Week 47 | 9 | 0 | No | | 0 |
| Week 102 | 7 | 0 | No | | 0 |
| Week 121 | 45 | 67.2 | Yes | 1 | 1 |
| Week 127 | 86 | 132 | Yes | 1 | 2 |

| **Feline-9** | | | | | |
|---|---|---|---|---|---|
| Week 0 | 20 | 27 | Yes | 1 | 1 |
| Week 41 | 19 | 20.5 | Yes | 1 | 1 |
| Week 98 | 33 | 28.1 | Yes | 1 | 1 |
| Week 115 | 40 | 39.3 | Yes | 1 | 1 |
| Week 119 | 31 | 95.8 | Yes | 0 | 3 |

| **Feline-10** | | | | | |
|---|---|---|---|---|---|
| Week 0 | 20 | 17.7 | Yes | 1 | 1 |
| Week 41 | 17 | 6.56 | Yes | 3 | 0 |
| Week 98 | 17 | 11.6 | Yes | 1 | 1 |
| Week 115 | 19 | 8.54 | Yes | 2 | 0 |
| Week 125 | 17 | 12.2 | Yes | 1 | 1 |
| Week 131 | 16 | 12.4 | Yes | 1 | 1 |
| Week 139 | 16 | 14.9 | Yes | 1 | 1 |
| * Week 0 = first sample | | | | | |

The upper reference limit for the concentration of SDMA [SDMA] in felines is 14 ug/dL, which represents the 95^{th} percentile of the concentration of SDMA in healthy subjects. *See* WO2015/035115.

Elevated BAIB levels are a marker of severity of kidney structural damage and are prognostic of mortality. [SDMA]/[BAIB] ratios below about 7 indicate functional as well as structural damage in kidney disease. [BAIB]/[SDMA] ratios above about 0.15 also indicate the functional loss with structural damage in kidney disease. Higher [BAIB]/[SDMA] ratios reflects an increased likelihood of early death, wherein lower [BAIB]/[SDMA] ratios reflect a decreased likelihood of early death. The reference limit for the [SDMA]/[BAIB] ratio in felines is 7. The reference limit for the [BAIB]/[SDMA] ratio in felines is 0.15.

### Example 3: BAIB in Felines With Kidney Stones

Serum levels of BAIB were determined at various times for feline subjects suffering from kidney stones. Elevated BAIB serum concentration was indicative of kidney stones as shown in Table 3

**Table 3**

| **ID** | **Time (weeks)** | **BAIB (µg/dL)** | **Diagnosis** | **Comment** |
|---|---|---|---|---|
| Feline-A | 0 | 20 | Oxalate stones/ Glomerulonephritis | |
| Feline-A | 8 | 117 | 100% calcium oxalate monohydrate | Deceased Week 56 |
| Feline-B | 0 | 9 | | |
| Feline-B | 24 | 32 | 100% calcium oxalate monohydrate Renal Stones | Left Kidney stone week 116 |
| | | | | Kidney failure Week 129 |
| Feline-C | 0 | 16 | Oxalate crystals | |
| Feline-C | 2 | 12 | Oxalate crystals | |
| Feline-D | 0 | 33 | Kidney stones / Acute Failure | |
| Feline-D | 12 | 219 | 100% calcium oxalate monohydrate | Deceased |
| Feline-E | 0 | 26 | Oxalate Kidney stones | |
| Feline-F | 0 | 8 | Small kidney stones (radiograph), January 2010 | |
| Feline-F | 160 | 16 | Renal Stones; 100% calcium oxalate monohydrate | |
| Feline-G | 0 | 20 | Oxalate stones/Kidney failure/Urolithasis | |
| Feline-G | 8 | 57 | US Rt kidney shows large stones present; also had x-rays same day with white shades present; Calcium oxylate dihydrate and monohydrate Week 8 | Deceased week 8 |
| Feline-H | 0 | 13 | Struvite/Oxalate Week 8 | |
| Feline-I | 0 | 14.1 | | |
| Feline-I | 26 | 16.4 | Renal, bilateral and bladder stones | |
| Feline-I | 86 | 13.7 | No special diet after diagnosed with stones | |
| Feline-I | 138 | 13.4 | Fed multiple commercial brands before stone dx | |
| Feline-I | 203 | 463.5 | 100% calcium oxalate monohydrate | Deceased Week 203 |
| Feline-J | 0 | 14.9 | | |
| Feline-J | 41 | 18.6 | Left kidney oxalate stones | |
| Feline-J | 104 | 25.0 | | |
| Feline-J | 185 | 24.0 | Scarring and fibrosis seen at necropsy | |
| Feline-K | 0 | 1.631 | | |
| Feline-K | 112 | 1.5 | Left kidney few small stones (struvite and not oxalate) | |
| Feline-K | 71 | 1.3 | | |
| Feline-K | 225 | 1.8 | | |
| Feline-L | 0 | 14.9 | calcium oxylate monohydrate 20%; misc material 80% | |
| Feline-L | 56 | 23.0 | | |
| Feline-L | 115 | 13.3 | | |
| Feline-L | 165 | 19.7 | | |
| Feline-L | 214 | 15.8 | Bilateral oxalate kidney stones Rt more than left | |
| Feline-M | 0 | 21.7 | | |
| Feline-M | 80 | 22.5 | | |
| Feline-M | 133 | 20.7 | | |
| Feline-M | 137 | 23.3 | | |
| Feline-M | 178 | 16.8 | Renal Stones; 5% calcium oxalate monohydrate, 95% miscellaneous material | Deceased Week 178 |
| Feline-N | 0 | 16.1 | | |
| Feline-N | 24 | 14.0 | | |
| Feline-N | 80 | 10.4 | | |
| Feline-N | 147 | 15.6 | | |
| Feline-N | 194 | 31.4 | | |
| Feline-N | 217 | 22.6 | Urolithiasis; 100% calcium oxalate monohydrate | Deceased Week 217 |
| Feline-O | 0 | 23.3 | | |
| Feline-O | 61 | 33.7 | | |
| Feline-O | 123 | 24.29 | Rt kidney oxalate stone | |
| | | | Week 230 | |
| Feline-P | 0 | 2.153 | | |
| Feline-P | 28 | 2.614 | | |
| Feline-P | 81 | 1.544 | | |
| Feline-P | 152 | 1.376 | | |
| Feline-P | 175 | 1.0 | Only 5% potentially calcium oxalate monohydrate (not certain), 95% miscellaneous material | Deceased Week 175 |
| Feline-Q | 0 | 14.3 | 12/17/2014 Lf kidney enlarged with oxalate stone; Rt kidney is normal to small | |
| Feline-Q | 88 | 11.2 | | |
| Feline-Q | 129 | 16.5 | | |
| Feline-R | 0 | 1.1 | | |
| Feline-R | 86 | 1.5 | | |
| Feline-R | 152 | 1.2 | | |
| Feline-R | 202 | 1.3 | | |
| Feline-R | 244 | 3.6 | Renal Stone; 100% calcium oxalate monohydrate | Deceased week 244 |
| Feline-S | 0 | 1.8 | Lf Kidney stones (not characterized and may not be oxalate) Week 152 | |
| Feline-S | 79 | 1.3 | Rt kidney stones (not characterized and may not be oxalate) week 192 | |
| Feline-S | 115 | 1.767 | Poor appetite (weeks 150-153) . | |
| | | | Weight loss (weeks 162-165). | |
| | | | Put on high fat diet. | |
| Feline-U | 0 | 15.09 | | |
| Feline-U | 52 | 19.67 | | |
| Feline-U | 75 | 18.57 | | |
| Feline-U | 124 | 22.73 | | |
| Feline-U | 128 | 33.15 | | |
| Feline-U | 213 | 46.55 | Renal Stones; 75% calcium oxalate monohydrate, 25% miscellaneous material | Deceased week 213 |
| Feline-V | 0 | 0.84 | | |
| Feline-V | 56 | 2.01 | | |
| Feline-V | 143 | 1.69 | | |
| Feline-V | 195 | 2.43 | | |
| Feline-V | 215 | 1.37 | Renal Stones; 100% calcium oxalate monohydrate | Deceased week 215 |
| Feline-W | 0 | | Jan 3 2014 Possible ureter or bladder stone. | |
| Feline-W | 31 | | Grossly normal kidneys on necropsy. | |
| Feline-W | 46 | 12 | 1/p inflammation in kidneys with medullary mineralization. | |
| Feline-Z | 0 | 0.98 | | |
| Feline-Z | 56 | 1.75 | | |
| Feline-Z | 115 | 1.47 | | |
| Feline-Z | 163 | 1.2 | | |
| Feline-Z | 183 | 0.98 | Renal Stones; 100% calcium oxalate monohydrate | Deceased week 183 |
| Feline-AA | 0 | 1.0 | | |
| Feline-AA | 24 | 1.0 | x-rays show left kidney stones, fever, and pyelonephritis. | |
| Feline-AA | 77 | 1.8 | Laser therapy | |
| Feline-AA | 127 | 1.3 | now has bilateral kidney stones. | |
| Feline-AA | 179 | 2.0 | | |
| Feline-AA | 254 | 140.1 | Bilateral oxalate stones | |
| Feline-AC | 0 | 15.0 | | |
| Feline-AC | 56 | 17.5 | | |
| Feline-AC | 118 | 13.4 | | |
| Feline-AC | 163 | 13.7 | | |
| Feline-AC | 220 | 12.9 | | |
| Feline-AC | 231 | 16.8 | Oxalate renal stones at | |
| | | | necropsy | |
| Feline-AD | 0 | 26 | Oxalate stones | |
| Feline-AE | 0 | 16.6 | | |
| Feline-AE | 20 | 20.5 | Renal or Bladder stones; 100% calcium oxalate monohydrateNecropsy - Bladder stone. | Deceased week 20 |
| Feline-AF | 0 | 2.1 | Urolithiasis/Bladder stones; 100% calcium oxalate monohydrate | |
| Feline-AG | 0 | 17.7 | | |
| Feline-AG | 56 | 20.1 | | |
| Feline-AG | 118 | 10.0 | | |
| Feline-AG | 146 | 18.4 | | |
| Feline-AG | 232 | 21.3 | Renal Stones; 100% calcium oxalate and phosphate apatite form Necropsy -Rt kidney stones. | Deceased week 232 |
| Feline-AH | 0 | 20.2 | | |
| Feline-AH | 32 | 23.0 | | |
| Feline-AH | 74 | 16.0 | | |
| Feline-AH | 110 | 74.1 | Renal Stones; 100% calcium oxalate monohydrate | Deceased week 110 |
| Feline-AI | 0 | 18.3 | | |
| Feline-AI | 54 | 23.2 | | |
| Feline-AI | 117 | 35.7 | | |
| Feline-AI | 177 | 23.0 | | |
| Feline-AI | 223 | 13.8 | Renal Stones; 100% calcium oxalate monohydrate | Deceased week 223 |
| Feline-AJ | 0 | 14.1 | | |
| Feline-AJ | 28 | 17.5 | | |
| Feline-AJ | 81 | 37.1 | | |
| Feline-AJ | 135 | 22.9 | | |
| Feline-AJ | 145 | 33.4 | Renal Stones; 95% calcium oxalate monohydrate, 5% calcium oxalate didydrate | Deceased week 146 |
| Feline-AK | 0 | 0.9 | X rays show Bladder and Rt kidney stones | |
| Feline-AK | 84 | 1.0 | | |
| Feline-AK | 140 | 0.9 | | |
| Feline-AK | 190 | 0.6 | | |
| Feline-AK | 214 | | | |
| Feline-AK | 263 | 1.6 | Urolithiasis/Bladder stones; 100% calcium oxalate monohydrate | |
| * Week 0 = first sample | | | | |

### Example 4: BAIB Levels in feline subjects suffering from glomerulonephritis (comparative example, not part of the invention)

Four feline patients and one canine patient died as a result of kidney disease. After the patients' death, necropsy resulted in a diagnosis of glomerulonephritis. SDMA, BAIB and CRE were measured (as described above) retrospectively in banked samples collected prior to the patients' death. BAIB was elevated in these patients as shown in Table 4

**Table 4**

| **Sample Name** | **SDMA** | **BAIB** | **CRE** |
|---|---|---|---|
| Feline-1 | 40 | 95.8 | 1.99 |
| Feline-2 | 86 | 132 | 2.49 |
| Feline-3 | 42 | 68.5 | 2.64 |
| Feline-4 | 20 | 117 | 1.96 |
| Canine-1 | 24 | 52 | 1.12 |

### Example 5: BAIB Levels in Canines with Kidney Disorder.

BAIB reference levels in normal canine subjects were determined in 136 dogs of both sexes, and from various breeds. Serum samples were collected and analyzed by LC-MS as described above. The upper reference limit based on the 95th percentile for this population was 1.24 µg/dL.

The presence of a discordance between serum SDMA and serum CRE concentrations (i.e., a high ratio of [SDMA]/[CRE] and serum SDMA above normal) is known to be indicative of a risk of premature death. *See* WO2015/035155. Accordingly, serum BAIB, CRE and SDMA were measured in 13 dogs with various levels of SDMA and CRE. The ratio [BAIB]/[SDMA] was calculated. These data are presented in Table 5.

**Table 5**

| **Dog** | **SDMA (µg/dL)** | **CRE (mg/dL)** | **BAIB (µg/dL)** | **SDMA : CRE Discordance*** | **[BAIB]/[SDMA]** |
|---|---|---|---|---|---|
| **Canine-1** | 14 | 1.7 | 0 | No | 0 |
| **Canine-2** | 12 | 1.6 | 0 | No | 0 |
| **Canine-3** | 7 | 0.5 | 0 | No | 0 |
| **Canine-4** | 12 | 1.4 | 0 | No | 0 |
| **Canine-5** | 10 | 1.6 | 0 | No | 0 |
| **Canine-6** | 56 | 7.7 | 1 | No | 0.0 |
| **Canine-7** | 21 | 2.4 | 0 | No | 0.0 |
| **Canine-8** | 20 | 8.6 | 4 | No | 0.2 |
| **Canine-9** | 21 | 2.2 | 96.1 | Yes | 4.6 |
| **Canine-10** | 50 | 1.4 | 77.1 | Yes | 1.5 |
| **Canine-11** | 21 | 1.6 | 24.1 | Yes | 1.1 |
| **Canine-12** | 84 | 1.6 | 30 | Yes | 0.4 |
| **Canine-13** | 64 | 5.4 | 91.3 | Yes | 1.4 |

| | | | | | |
|---|---|---|---|---|---|
| * [SDMA]/[CRE] > 10 and SDMA > 14 µg/dL | | | | | |

The canine cohort was subsequently monitored. In dogs exhibiting [SDMA]:[CRE] discordance, the BAIB was elevated above the normal cutoff value. In dogs exhibiting [SDMA]:[CRE] discordance, the [BAIB]/[SDMA] ratio was elevated relative to the dogs not exhibiting the discordance. On follow up, the dogs exhibiting [SDMA]:[CRE] discordance were reported deceased. A ratio of [BAIB]/[SDMA] greater than 1.5 was determined as indicative of kidney dysfunction and a risk of premature death. An elevated serum concentration of BAIB is indicative of kidney dysfunction and a risk of premature death

### Example 6: Measurement of BAIB and SDMA in Cancer Patients

BAIB and SDMA were measured in serum of seven canine and six feline patients presenting to local clinics with a variety of cancers.

Of the seven canine cancer patients, only one (C6) had BAIB levels above the upper limit of the normal reference range (3.6 pg/mL). Canine cancer patient C6 was found to have metastatic renal carcinoma, i.e., C6 was the only canine in the group where the cancer had spread to the kidneys.

Of the six feline cancer patients, three patients with positive kidney histopathological findings (metastatic renal carcinoma, interstitial lymphoplasmacytic nephritis, inflammation, fibrosis) had elevated levels of BAIB (F3, F4 and F5) above the reference range (2.0 µg/dl). The three patients with normal kidney histopathology had BAIB levels within the normal reference.

**Table 6**

| **Species** | **Patient ID** | **SDMA (ug/dL)** | **BAIB (ug/dL)** | **Kidney Histopathology** |
|---|---|---|---|---|
| Canine | C1 | 10 | 1 | No findings |
| Canine | C2 | 10 | 0 | No findings |
| Canine | C3 | 11 | 1 | No findings |
| Canine | C4 | 12 | < 0.39 | Normal kidneys |
| Canine | C5 | 16 | 1 | Abnormal kidneys, Multifocal renal tubular ectasia (incidental finding) |
| Canine | C6 | 17 | 59 | Metastatic renal carcinoma |
| Canine | C7 | 8 | 0 | Interstitial lymphoplasmacytic nephritis, multifocal, inflammation |
| feline | F1 | 12 | 1 | No findings |
| Feline | F2 | 11 | 1 | No findings |
| Feline | F3 | 15 | 41 | Metastatic renal carcinoma |
| Feline | F4 | 11 | 32 | Inflammation with fibrosis |
| Feline | F5 | 17 | 18 | Rough surface and enlarged medullas; Extremely thickened and inflamed cortex |
| Feline | F6 | 11 | 1 | No findings |

### Example 7: Preparation of BAIB-G6PHD Conjugate

Conjugates of BAIB and G6PDH were prepared by conjugating the BAIB analog SDMA-SH with G6PDH activated with SIA in the presence of NAD and G6P.

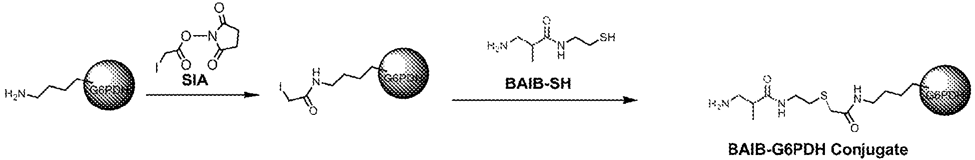

**Enzyme Preactivation with SIA:** One vial of Glucose-6-phosphate dehydrogenase (G6PDH) (12 mg) was dissolved in 3 mL MES buffer (50 mM, pH8 .0) and rotated for 1 hour to ensure that the enzyme is fully dissolved. The enzyme solution was kept on ice until needed. An additional 4.5 mL MES buffer (50 mM, pH 8.0) was added to the enzyme solution, mixed well through vortexing (5 seconds) and keep the solution on ice for 10 minutes. 100mg G6P was dissolve in 1mL deionized water and on ice for 10 min. 200 mg NADH was dissolve in 1 mL deionized water and keep on ice for 10 min. 0.68 mL G6P solution and 0.34 mL NADH solution were added to the enzyme solution, mixed well through vortexing (5 seconds) and kept on ice for 10 min. One vial of SIA (50 mg) was dissolved in 0.5 mL DMSO (100 mg/mL). 0.14 mL of the SIA solution was added to the enzyme solution, mixed well through vortexing (5 seconds), covered with aluminum foil, and rotated at room temperature for 2 hours. The solution was transferred to a G2 Slide-A-Lyzer Dialysis Cassette and dialyzed for five hours against PBS buffer (4 L) at 4 °C in the dark. The buffer was changed to fresh PBS (4 L) and the solution was dialyzed at 4 °C overnight in the dark. The dialysis buffer was changed to MES (4L, 25mM, pH 8.0) and the solution was dialyzed for 3 hours at 4 °C. 12.5mL of the enzyme solution was removed from the dialysis cassette and 0.32mL MES buffer (1M, pH8.0) and 0.32 mL EDTA ( 0.2M, pH8.0) was added to bring the final concentration of the solution to 50mM MES and 5mM EDTA. If necessary, if the enzyme solution is less than 12.5mL, the volumes of MES and EDTA may be adjusted accordingly. The solution was degassed with argon for 5 minutes.

BAIB-SH analog (Carboxyl end) was prepared as described in Example 12 and coupled to the activated enzyme as follows: BAIB-SH (400 eq, 0.096 mmol, 15.6 mg) was dissolved in water (0.156 ml) and was added to SIA-activated G6PDH enzyme solution. The reaction was stirred at 4 °C for 36 hrs. The BAIB-G6PDH conjugate was formed and purified by dialysis against PBS (4L) for 6 hrs at 4 °C with three times buffer change.

### Example 8: Preparation of Glutaraldehyde-BAIB conjugate

A glutaraldehyde-BAIB conjugate was prepared with 2 mg/mL BAIB in 20mM PBS with 0.15M NaCl (5 mL, 10 mg total) with 25 µL (6.25 mg) of glutaraldehyde added (25% in water). After reacted at room temperature for 1 hr, NaBH4 (4 eq of BAIB, 0.776 mmol, 48 mg) was added and the reaction mixture was stirred at 4 C for 18 hrs.

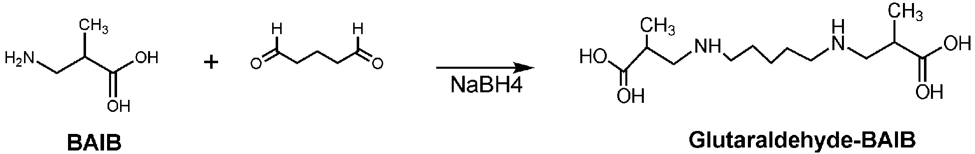

### Example 9: Preparation of poly-lysine-BAIB conjugate

A poly-lysine-BAIB conjugate was prepared according the following reaction scheme to a concentration of 0.1mg/mL in water.

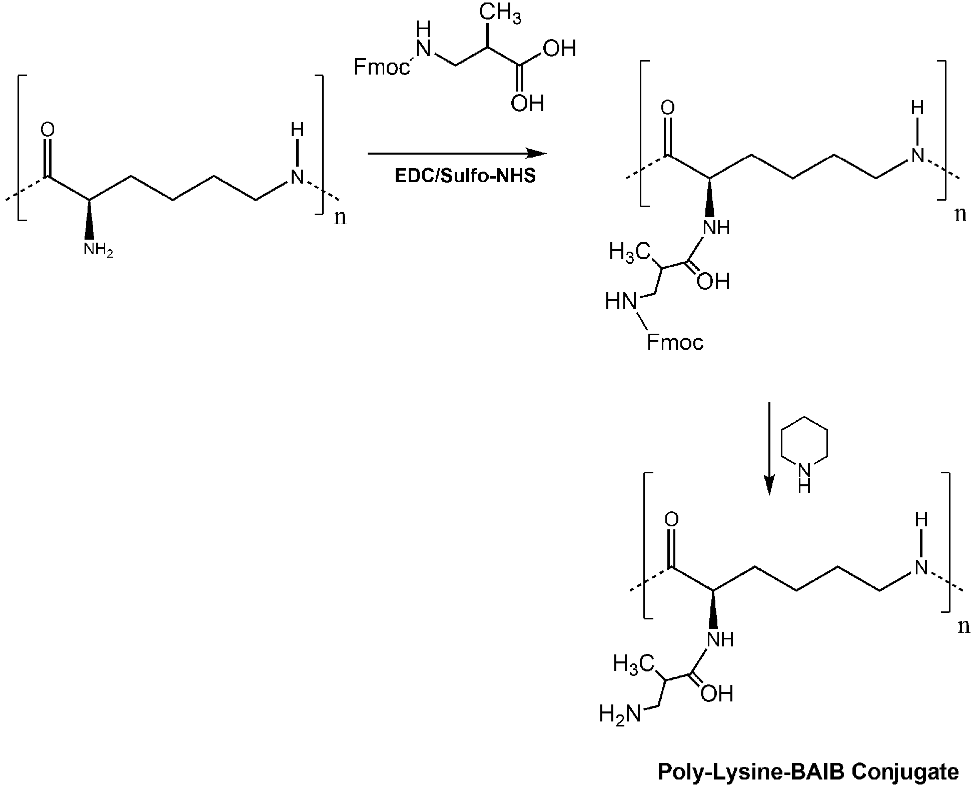

Fmoc-BAIB (5 mg) was dissolved in water (5 mL) and was added EDC (2 mg) and Sulfo-NHS(3 mg). The solution was stirred at room temperature for 15 min and then poly-lysine solution (20 ml, 0.1%) was added and then the mixture was stirred for 3 hours at room temperature. The solution was added piperdine to final concentration at 20% and incubated at least 1 hour at room temperature. The reaction solution was then dialyzed in a 10K MWCO dialysis cassette (30 ml) against PBS (4L) at 4 °C and change the buffer twice..

### Example 10: Preparation of Particle-BAIB Conjugate

0.4 mL of particles at 5% solids were mixed with 0.8mL BAIB at 5 mg/mL concentration in 2.8mLs 50mM Phosphate buffer and mixed end-over-end at 4 °C for two days.

### Example 11: Synthesis of a BAIB Thiol (amine end) and BSA-Conjugated BAIB.

An exemplary immunogen, BAIB (amine end)-BSA conjugate, was prepared according to the following procedure.

First, a BAIB-SH analog (amino end) was prepared in accordance with the following reaction scheme and method described below. 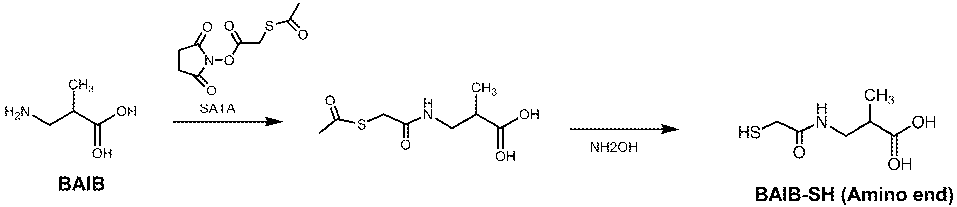
1. 22 mg (0.216 mmol) of BAIB (Sigma-Aldrich Cat. No. 217794) was dissolved in PBS (4 mL).
2. 50 mg (0.216 mmol) SATA (N-succinimidyl S-acetylthioacetate -- Thermo Catalog No. PD199377) was dissolved in DMSO (0.4 mL) and added to the BAIB solution.
3. The reaction was inverted for 30 minutes at 20 °C.
4. The reaction was then treated with a deacetylation solution (0.9 mL, 0.5 M hydroxylamine, 25 mM EDTA in PBS, pH 7.3).
5. The reaction was inverted for 4 hours at 4 °C to provide BAIB-SH (amino end).

The BAIB-SH was coupled to BSA as follows:
1. 1 mL of the reaction solution was added to a vial of maleimide-activated BSA (5 mg) (Sigma-Aldrich Cat. No. 054M4801V) and the reaction was inverted for 18 hours at 2 °C.
3. The conjugate was placed into a 10 MW cut off, 3 mL dialysis cassette and dialyzed against PBS (4 L) at 4 °C for 48 hours.
4. Coupling efficiency was determined by Ellman's test according to methods known in the art using excess thiol solution from step 6 above: 10 µL of sample + 20 µL Ellman's reagent + 70 µL of DTNB buffer then read at 412 nm.

### Example 12: Synthesis of a BAIB Thiol (carboxy end) and BSA-conjugated BAIB.

An exemplary immunogen, BAIB (carboxy end)-BSA conjugate, was prepared according to the following procedure.

### BAIB Resin Preparation:

BAIB-SH Resin was prepared according the following general reaction scheme and as described below. 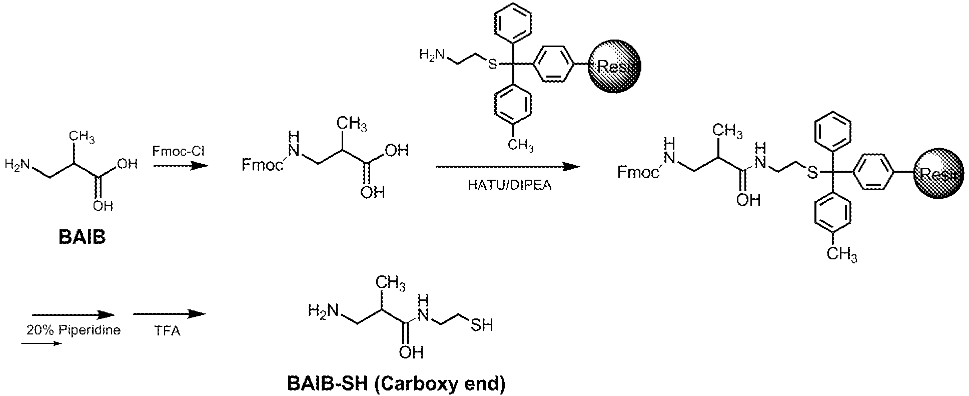
1. A mixture comprising 150 mg (0.28 mmol) BAIB (Sigma-Aldrich Cat. No. 217794), 400 mg (0.31 mmol) Fmoc-Cl (Fluka BCBH6153V), 0.41 mL (0.47 mmol) DIPEA (Sigma-Aldrich Cat. No. 77996JJ), and 18 mL DMF (Sigma-Aldrich Cat. No. 23185) was inverted for 3 hours at 20C.
2. The following were added to the mixture above: 1.2 g (0.093 mmol) 4-methoxy trityl resin (Novabiochem S6013887 348) and 530 mg (0.28 mmol) HATU (Novabiochem S8446513 309). The resulting solution was inverted for a further 18 hours at 20 °C.
3. 20% piperidine in DMF was then added and inverted for 15 min (3 x 18 mL).
3. Following incubation, the resin was washed with DMF (4 x 18 mL), then MeOH (4 x 18 mL), and dried to give 0.95 g BAIB-SH resin.

### Synthesis of BAIB (carboxy end)-BSA conjugate

1. 200 mg of BAIB-SH resin was added to 4 mL of TFA (Sigma-Aldrich Cat. No. 91707).
2. The BAIB-TFA mixture was inverted at 20 °C for 1 hr.
3. The reaction was dried to give 20 mg of the BAIB-thiol analog.
4. The thiol from step 3 was dissolved in PBS (4 mL).
6. The thiol solution was added to a fours vials of maleimide activated BSA (20mg) (Sigma-Aldrich Cat. No. 054M4801V) and the reaction was inverted for 18 hours at 4 °C. Excess solution was reserved for Ellman's test.: 10 µL of sample + 20µL Ellman's reagent + 70µL of DTNB buffer then read at 412nm.
7. The conjugate was placed into a 10MW cut off, 3 mL dialysis cassette and dialyzed against PBS (4 L) at 4 °C for 48 hours.
8. Coupling efficiency was determined by Ellman's test according to methods known in the art using excess thiol solution from step 5 above: 10 µL of sample + 20µL Ellman's reagent + 70µL of DTNB buffer then read at 412nm.

### Example 13: Synthesis of KLH-conjugated BAIB.

An exemplary immunogen, BAIB (carboxy end)-KLH conjugate, was prepared according to the following procedure:
1. 100 mg of BAIB-SH resin (see Example 11 above) was added to 2 mL of TFA (Sigma-Aldrich Cat. No. SHBD1537V).
2. The BAIB-TFA mixture was inverted at 20 °C for 1 hour, and the resin was filtered off and washed with 0.5 mL of acetonitrile.
3. The reaction was dried to give 15 mg of BAIB-SH as a clear oil.
4. The oil from step 3 was dissolved in PBS (3 mL) to form a BAIB thiol solution.
5. 2 mL of the thiol solution was added to 10 mg of maleimide activated KLH (Sigma-Aldrich Cat. No. 072M4796) and the reaction was inverted for 18 hours at 4 °C.
6. The conjugate was placed into a 10 MW cut off, 3 mL dialysis cassette and dialyzed against PBS (4 L) at 4 °C for 48 hours.
7. Coupling efficiency was determined by Ellman's test according to methods known in the art using excess thiol solution from step 5 above.: 10 µL of sample + 20µL Ellman's reagent + 70µL of DTNB buffer then read at 412nm.

### Example 14: Method for generating anti-BAIB polyclonal antibodies

The immunization protocol for generating the anti-BAIB polyclonal antibodies may be carried out according to the following protocol, which is well known to those skilled in the art. Rabbits are immunized with, for example, one of the immunogens from Examples 8-12 above, or another BAIB specific antigen. In each case, exemplary immunizations are performed by injecting 0.5 mg of the immunogen in 1 mL of phosphate buffered saline (PBS) mixed with 1 mL of Freund's complete adjuvant. Each animal may receive 20-30 intradermal injections on their shaved back. Each animal may be boosted with 0.25 mg of immunogen in 1 mL PBS mixed with equal volume of Freund's incomplete adjuvant in the hind legs. The boosting shots may be given each month after the primary injection. Test bleeds of 5 mL blood can be taken from each rabbit 7-10 days after each boost. Production bleeds of 40 mL can be taken from each rabbit after the third booster shot, when the antisera titer was greater than about 1:2000. Antiserum titer is the dilution of antiserum that generates the steepest calibration curve for the assay.

### Example 15: Preparation of anti-BAIB monoclonal antibodies

Methods of producing monoclonal antibodies are within the skill in the art. In embodiments, the antibody is a monoclonal antibody raised against glutaraldehyde-BAIB conjugate, poly-lysine-BAIB conjugate, BAIB (amine end)-BSA conjugate, BAIB (carboxy end)-BSA conjugate, BAIB (amine end)-KLH conjugate, or BAIB (carboxy end)-KLH conjugate. Anti-bodies may be purified and characterized using methods well known to those of skill in the art.

### Example 16: Preparation of anti-rabbit BAIB anti-sera

Anti-BAIB antibody specificity was shown with a binding assay using a rabbit anti-BAIB serum raised against a mixture of BAIB(amine end)-KLH conjugate and BAIB(Carboxy end)-KLH conjugate. BAIB was weighed and dissolved in PBS, pH 7.4 to 1.0 mg/ml and further diluted to 50 µg/ml. Protein A (rPA) slurry was incubated with rabbit anti-BAIB antiserum to capture IgG. In the negative control, Protein A (rPA) slurry was incubated with PBS. Following the incubations, six hundred (600) µL of the rPA slurry was added to micro spin columns and spun to remove liquid. Three hundred (300) µL of 50 µg/mL BAIB was added to the columns and incubated for 100 minutes at 25 °C. The columns were then spun and the flow through collected. The flow through was then prepared for analysis by LC-MS as follows. Fifty (50) µL of each flow through sample was mixed with 50 µL of BAIB Internal Standard in a 96 well plate, then centrifuged at 3,000 x g for 20 minutes. 300 µL of Acetonitrile was then added to each sample in the plate, and the plate was then sonicated for 20 minutes. The plate was then centrifuged at 3,000 x g for 20 minutes. 300 µL of the supernatant was then filtered by loading into a 96 well oplate filter (0.45um) and centrifuged at 3,000 x g for 20 minutes. Following filtration, the plate was then dried for 4 hours in a SpeedVac. Post drying, 50 µL of a 20% Acetonitrile solution was added to each sample. The samples were then run in LC_MS. As shown below in Table 7, the anti-BAIB:rProtA column bound 6.6% more BAIB than the control rProtA column.

**Table 7**

| **Condition** | **BAIB Original** | **rProtA column** | **anti-BAIB:rProtAcolumn** |
|---|---|---|---|
| Initial BAIB ug | 18.2 | 17.2 | 15.6 |
| [BAIB] Flow Through | | 9.07 | 7.81 |
| % Flow Through | | 49 | 43 |
| % Bound | | 51 | 57 |
| % Specific Binding | | | 6.6 |

Any numerical values recited herein include all values from the lower value to the upper value in increments of one unit provided that there is a separation of at least two units between any lower value and any higher value. As an example, if it is stated that the concentration of a component or value of a process variable such as, for example, size, angle size, pressure, time and the like, is, for example, from 1 to 90, specifically from 20 to 80, more specifically from 30 to 70, it is intended that values such as 15 to 85, 22 to 68, 43 to 51, 30 to 32, etc. are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner.

## Claims

1. An *in vitro* method for determining whether a canine or feline animal is suffering from kidney disease as the result of structural damage or glomerulonephritis, the method comprising measuring β-aminoisobutyric acid (BAIB) in a urine sample or a blood sample from the animal and determining kidney disease based upon the concentration of BAIB in the sample, further comprising comparing the concentration of BAIB in the sample to a reference concentration related to the concentration in BAIB in samples from healthy animals,
wherein the measuring comprises contacting the sample with an anti-BAIB antibody and determining binding or the amount of binding between the antibody and BAIB in the sample,
further wherein the anti-BAIB antibody is raised against a conjugate comprising BAIB and a carrier protein, BAIB and polylysine, or BAIB and glutaraldehyde and wherein the structural damage is the result of inflammation, fibrosis, injury, kidney stones or infiltration by cancer.

2. An *in vitro* method of diagnosing a kidney disease which is the result of structural damage or glomerulonephritis in a canine or feline animal subject, the method comprising:
contacting a urine or blood sample obtained from the subject with an anti-BAIB antibody and determining binding or the amount of binding between the antibody and BAIB in the sample, wherein the antibody is raised against a conjugate comprising BAIB and a carrier protein, BAIB and polylysine, or BAIB and glutaraldehyde;
measuring the concentration of BAIB in the sample; comparing the level of BAIB to a reference concentration of BAIB in healthy subjects;
and
diagnosing a kidney disorder which is a result of structural damage or glomerulonephritis where the value of BAIB in the sample is in excess of the reference concentration and wherein the structural damage is the result of inflammation, fibrosis, injury, kidney stones or infiltration by cancer.

3. An in vitro method for determining whether a canine or feline animal subject is suffering from kidney disease due to structural damage or glomerulonephritis, the method comprising:
measuring the concentration of BAIB and symmetrical dimethyl arginine (SDMA) in a urine or blood sample obtained from the subject;
wherein the measuring comprises contacting the sample with an anti-BAIB antibody raised against a conjugate comprising BAIB and a carrier protein, BAIB and polylysine, or BAIB and glutaraldehyde, and
determining binding or the amount of binding between the anti-BAIB and BAIB in the sample; and determining kidney disease when the ratio of the concentration of BAIB [BAIB] to concentration of SDMA [SDMA] is greater than 0.15 or when the ratio of [SDMA] to [BAIB] is less than 7 and wherein the structural damage is the result of inflammation, fibrosis, injury, kidney stones or infiltration by cancer.

4. An in vitro method of diagnosing a renal disorder which is a loss of kidney function due to structural damage in a canine or feline animal subject, the method comprising: measuring the concentration of one or more of BAIB and SDMA in a urine or blood sample obtained from the subject; wherein the measuring comprises contacting the sample with an anti-BAIB antibody raised against a conjugate of BAIB and a carrier protein, BAIB and polylysine, or BAIB and glutaraldehyde, and
determining binding or the amount of binding between the anti-BAIB and BAIB in the sample; comparing the level BAIB and SDMA to reference concentrations of BAIB and SDMA
in healthy subjects;
diagnosing loss of kidney function when the concentration of SDMA in the sample is in excess of the SDMA reference concentration and
diagnosing that the loss of kidney function is a result of structural damage when the concentration of BAIB in the sample is in excess of the BAIB reference concentration and wherein the structural damage is the result of inflammation, fibrosis, injury, kidney stones or infiltration by cancer.

5. The method of any of claims 1-4, wherein the reference concentration reflects
the 95^{th} percentile of the concentration of BAIB in healthy animals.

6. The method of claim 5, wherein the animal is a feline and the reference concentration is 2.0 µg/dL BAIB, or wherein the animal is a canine and the reference concentration is 1.2 µg/dL BAIB, and/or wherein the reference concentration for SDMA is 14 µg/dL.

7. The method of claim 1, wherein the carrier protein is preferably one of KLH and BSA.

8. An in vitro method of determining the presence or amount of BAIB in a sample, the method comprising contacting an antibody raised against a conjugate of BAIB with the sample and determining binding or the amount of binding between the antibody and BAIB in the sample, further comprising contacting the sample and the antibody with a conjugate comprising BAIB and a detectable label,
wherein the sample is a urine sample or a blood sample from a canine or feline animal suffering from kidney disease due to structural damage or glomerulonephritis,
further wherein the antibody is raised against a conjugate of BAIB and a carrier protein, BAIB and polylysine, or BAIB and glutaraldehyde, and wherein the structural damage is the result of inflammation, fibrosis, injury, kidney stones or infiltration by cancer .

9. The method according to any one of claims 1- 8, wherein the blood sample is serum or plasma.

10. The method according to claim 5, wherein at least one of the reference concentration of BAIB and the reference concentration of SDMA - if measured - reflects the 95th percentile of the concentration of BAIB and SDMA in samples from healthy animals.

11. The method according to claim 8, wherein the antibody comprises a label.

## Patentansprüche

1. *In-vitro*-Verfahren zur Bestimmung, ob ein Hunde- oder Katzentier an einer Nierenerkrankung als Folge einer strukturellen Schädigung oder Glomerulonephritis leidet, das Verfahren umfassend Messen von β-Aminoisobuttersäure ("β-aminoisobutyric acid", BAIB) in einer Urinprobe oder einer Blutprobe von dem Tier und Bestimmen einer Nierenerkrankung basierend auf der Konzentration von BAIB in der Probe, ferner umfassend Vergleichen der BAIB-Konzentration in der Probe mit einer Referenzkonzentration, die sich auf die BAIB-Konzentration in Proben von gesunden Tieren bezieht, wobei das Messen das Inkontaktbringen der Probe mit einem Anti-BAIB-Antikörper und Bestimmen der Bindung oder des Ausmaßes an Bindung zwischen dem Antikörper und BAIB in der Probe umfasst,
wobei der Anti-BAIB-Antikörper ferner gegen ein Konjugat umfassend BAIB und einem Trägerprotein, BAIB und Polylysin oder BAIB und Glutaraldehyd gerichtet ist und wobei die strukturelle Schädigung die Folge von Entzündung, Fibrose, Verletzung, Nierensteinen oder Infiltration durch Krebs ist.

2. *In-vitro*-Verfahren zum Diagnostizieren einer Nierenerkrankung, die die Folge von struktureller Schädigung oder Glomerulonephritis bei einem Hunde- oder eine Katzentiersubjekt ist, das Verfahren umfassend:
Inkontaktbringen einer Urin- oder Blutprobe, die von dem Subjekt mit einem Anti-BAIB-Antikörper erhalten wurde, und Bestimmen der Bindung oder des Ausmaßes an Bindung zwischen dem Antikörper und BAIB in der Probe, wobei der Antikörper gegen ein Konjugat, umfassend BAIB und einem Trägerprotein, BAIB und Polylysin oder BAIB und Glutaraldehyd, gerichtet ist;
Messen der Konzentration von BAIB in der Probe;
Vergleichen des BAIB-Spiegels mit einer Referenzkonzentration von BAIB in gesunden Subjekten; und
Diagnostizieren einer Nierenerkrankung, die eine Folge von struktureller Schädigung oder Glomerulonephritis ist, bei der der BAIB-Wert in der Probe über der Referenzkonzentration liegt und wobei die strukturelle Schädigung die Folge von Entzündung, Fibrose, Verletzung, Nierensteinen oder Infiltration durch Krebs ist.

3. In-vitro-Verfahren zum Bestimmen, ob ein Hunde- oder eine Katzentiersubjekt an einer Nierenerkrankung aufgrund von struktureller Schädigung oder Glomerulonephritis leidet, das Verfahren umfassend:
Messen der Konzentration von BAIB und symmetrischem Dimethylarginin ("symmetrical dimethyl arginine", SDMA) in einer Urin- oder Blutprobe, die von dem Subjekt erhalten wurde;
wobei das Messen das Inkontaktbringen der Probe mit einem Anti-BAIB-Antikörper, der gegen ein Konjugat, umfassend BAIB und ein Trägerprotein, BAIB und Polylysin oder BAIB und Glutaraldehyd, gerichtet ist, und Bestimmen der Bindung oder des Ausmaßes an Bindung zwischen dem Anti-BAIB und BAIB in der Probe; und
Bestimmen einer Nierenerkrankung, wenn das Verhältnis der Konzentration von BAIB [BAIB] zur Konzentration von SDMA [SDMA] größer als 0,15 ist oder wenn das Verhältnis von [SDMA] zu [BAIB] kleiner als 7 ist und wobei die strukturelle Schädigung die Folge von Entzündung, Fibrose, Verletzung, Nierensteinen oder Infiltration durch Krebs ist.

4. *In-vitro*-Verfahren zum Diagnostizieren einer Nierenstörung, die ein Verlust der Nierenfunktion aufgrund von struktureller Schädigung in einem Hund- oder Katzentiersubjekt ist, das Verfahren umfassend:
Messen der Konzentration von einem oder mehreren von BAIB und SDMA in einer Urin- oder Blutprobe, die von dem Subjekt erhalten wurde; wobei das Messen das Inkontaktbringen der Probe mit einem Anti-BAIB-Antikörper, der gegen ein Konjugat von BAIB und einem Trägerprotein, BAIB und Polylysin oder BAIB und Glutaraldehyd, gerichtet ist, umfasst, und
Bestimmen der Bindung oder des Ausmaßes an Bindung zwischen dem Anti-BAIB und BAIB in der Probe;
Vergleichen des BAIB- und SDMA-Spiegels mit Referenzkonzentrationen von BAIB und SDMA bei gesunden Subjekten;
Diagnostizieren des Verlusts der Nierenfunktion, wenn die Konzentration von SDMA in der Probe über der SDMA-Referenzkonzentration liegt und
Diagnostizieren, dass der Verlust der Nierenfunktion die Folge einer strukturellen Schädigung ist, wenn die Konzentration von BAIB in der Probe über der BAIB-Referenzkonzentration liegt, und wobei die strukturelle Schädigung die Folge von Entzündung, Fibrose, Verletzung, Nierensteinen oder einer Infiltration durch Krebs ist.

5. Verfahren gemäß einem der Ansprüche 1 bis 4, wobei die Referenzkonzentration das 95. Perzentil der Konzentration von BAIB in gesunden Tieren widerspiegelt.

6. Verfahren gemäß Anspruch 5, wobei das Tier eine Katze ist und die Referenzkonzentration 2,0 µg/dl BAIB ist, oder wobei das Tier ein Hund ist und die Referenzkonzentration 1,2 µg/dl BAIB ist, und/oder wobei die Referenzkonzentration für SDMA 14 µg/dl ist.

7. Verfahren gemäß Anspruch 1, wobei das Trägerprotein vorzugsweise eines von KLH und BSA ist.

8. *In-vitro*-Verfahren zur Bestimmung des Vorhandenseins oder der Menge von BAIB in einer Probe, das Verfahren umfassend das Inkontaktbringen eines Antikörpers, der gegen ein Konjugat von BAIB gerichtet ist, mit der Probe und Bestimmen der Bindung oder des Ausmaßes an Bindung zwischen dem Antikörper und BAIB in der Probe, ferner umfassend das Inkontaktbringen der Probe und des Antikörpers mit einem Konjugat, das BAIB und eine detektierbare Markierung umfasst,
wobei die Probe eine Urinprobe oder eine Blutprobe von einem Hunde- oder Katzentier ist, das an einer Nierenerkrankung durch strukturelle Schädigung oder Glomerulonephritis leidet,
wobei der Antikörper ferner gegen ein Konjugat aus BAIB und einem Trägerprotein, BAIB und Polylysin oder BAIB und Glutaraldehyd, gerichtet ist und wobei die strukturelle Schädigung die Folge von Entzündung, Fibrose, Verletzung, Nierensteinen oder Infiltration durch Krebs ist.

9. Verfahren gemäß irgendeinem der Ansprüche 1 bis 8, wobei die Blutprobe Serum oder Plasma ist.

10. Verfahren gemäß Anspruch 5, wobei mindestens eine der Referenzkonzentrationen von BAIB und der Referenzkonzentration von SDMA - falls gemessen - das 95. Perzentil der Konzentration von BAIB und SDMA in Proben von gesunden Tieren widerspiegelt.

11. Verfahren gemäß Anspruch 8, wobei der Antikörper eine Markierung umfasst.

## Revendications

1. Procédé *in vitro* pour déterminer si un animal canin ou félin souffre d'une maladie rénale résultant d'une lésion structurelle ou d'une glomérulonéphrite, le procédé comprenant la mesure de l'acide β-aminoisobutyrique (BAIB) dans un échantillon d'urine ou un échantillon de sang provenant de l'animal et la détermination d'une maladie rénale sur la base de la concentration en BAIB dans l'échantillon, comprenant en outre la comparaison de la concentration en BAIB dans l'échantillon à une concentration de référence associée à la concentration en BAIB dans des échantillons d'animaux en bonne santé,
dans lequel la mesure comprend la mise en contact de l'échantillon avec un anticorps anti-BAIB et la détermination de la liaison ou de la quantité de liaison entre l'anticorps et BAIB dans l'échantillon,
en outre dans lequel l'anticorps anti-BAIB est dirigé contre un conjugué comprenant BAIB et une protéine porteuse, BAIB et une polylysine, ou BAIB et un glutaraldéhyde et dans lequel la lésion structurelle est le résultat d'une inflammation, d'une fibrose, d'une blessure, de calculs rénaux ou d'une infiltration par un cancer.

2. Procédé *in vitro* de diagnostic d'une maladie rénale résultant d'une lésion structurelle ou d'une glomérulonéphrite chez un sujet animal canin ou félin, le procédé comprenant :
la mise en contact d'un échantillon d'urine ou de sang obtenu à partir du sujet avec un anticorps anti-BAIB et la détermination de la liaison ou de la quantité de liaison entre l'anticorps et BAIB dans l'échantillon, dans lequel l'anticorps est dirigé contre un conjugué comprenant BAIB et une protéine porteuse, BAIB et une polylysine, ou BAIB et un glutaraldéhyde ;
la mesure de la concentration en BAIB dans l'échantillon ; la comparaison du taux de BAIB à une concentration de référence en BAIB chez des sujets en bonne santé ;
et
le diagnostic d'un trouble rénal résultant d'une lésion structurelle ou d'une glomérulonéphrite lorsque la valeur de BAIB dans l'échantillon est supérieure à la concentration de référence et dans lequel la lésion structurelle est le résultat d'une inflammation, d'une fibrose, d'une blessure, de calculs rénaux ou d'une infiltration par un cancer.

3. Procédé in vitro pour déterminer si un sujet animal canin ou félin souffre d'une maladie rénale due à une lésion structurelle ou à une glomérulonéphrite, le procédé comprenant :
la mesure de la concentration en BAIB et en diméthylarginine symétrique (SDMA) dans un échantillon d'urine ou de sang obtenu à partir du sujet ;
dans lequel la mesure comprend la mise en contact de l'échantillon avec un anticorps anti-BAIB dirigé contre un conjugué comprenant BAIB et une protéine porteuse, BAIB et une polylysine, ou BAIB et un glutaraldéhyde, et
la détermination de la liaison ou de la quantité de liaison entre l'anti-BAIB et BAIB dans l'échantillon ; et la détermination de la maladie rénale lorsque le rapport concentration en BAIB [BAIB] sur concentration en SDMA [SDMA] est supérieur à 0,15 ou lorsque le rapport [SDMA] sur [BAIB] est inférieur à 7 et dans lequel la lésion structurelle est le résultat d'une inflammation, d'une fibrose, d'une blessure, de calculs rénaux ou d'une infiltration par un cancer.

4. Procédé in vitro de diagnostic d'un trouble rénal qui est une perte de fonction rénale due à une lésion structurelle chez un sujet animal canin ou félin, le procédé comprenant : la mesure de la concentration d'un ou de plusieurs parmi BAIB et SDMA dans un échantillon d'urine ou de sang obtenu à partir du sujet ; dans lequel la mesure comprend la mise en contact de l'échantillon avec un anticorps anti-BAIB dirigé contre un conjugué de BAIB et d'une protéine porteuse, de BAIB et d'une polylysine, ou de BAIB et d'un glutaraldéhyde, et
la détermination de la liaison ou de la quantité de liaison entre l'anti-BAIB et BAIB dans l'échantillon ; la comparaison des taux de BAIB et de SDMA à des concentrations de référence de BAIB et de SDMA chez des sujets en bonne santé ;
le diagnostic d'une perte de la fonction rénale lorsque la concentration en SDMA dans l'échantillon est supérieure à la concentration de référence en SDMA et
le diagnostic que la perte de la fonction rénale résulte d'une lésion structurelle lorsque la concentration en BAIB dans l'échantillon est supérieure à la concentration de référence en BAIB et dans lequel la lésion structurelle est le résultat d'une inflammation, d'une fibrose, d'une blessure, de calculs rénaux ou d'une infiltration par un cancer.

5. Procédé selon l'une quelconque des revendications 1 à 4, dans laquelle la concentration de référence reflète le 95^{e} percentile de la concentration en BAIB chez des animaux en bonne santé.

6. Procédé selon la revendication 5, dans lequel l'animal est un félin et la concentration de référence est de 2,0 µg/dl de BAIB, ou dans lequel l'animal est un canin et la concentration de référence est de 1,2 µg/dl de BAIB, et/ou dans lequel la concentration de référence pour SDMA est de 14 µg/dl.

7. Procédé selon la revendication 1, dans lequel la protéine porteuse est de préférence l'une parmi KLH et BSA.

8. Procédé in vitro de détermination de la présence ou de la quantité de BAIB dans un échantillon, le procédé comprenant la mise en contact d'un anticorps dirigé contre un conjugué de BAIB avec l'échantillon et la détermination de la liaison ou de la quantité de liaison entre l'anticorps et BAIB dans l'échantillon, comprenant en outre la mise en contact de l'échantillon et de l'anticorps avec un conjugué comprenant BAIB et un marqueur détectable,
dans lequel l'échantillon est un échantillon d'urine ou un échantillon de sang d'un animal canin ou félin souffrant d'une maladie rénale due à une lésion structurelle ou à une glomérulonéphrite,
en outre dans lequel l'anticorps est dirigé contre un conjugué de BAIB et d'une protéine porteuse, de BAIB et d'une polylysine, ou de BAIB et d'un glutaraldéhyde, et dans lequel la lésion structurelle est le résultat d'une inflammation, d'une fibrose, d'une blessure, de calculs rénaux ou d'une infiltration par un cancer.

9. Procédé selon l'une quelconque des revendications 1 à 8, dans lequel l'échantillon de sang est du sérum ou du plasma.

10. Procédé selon la revendication 5, dans lequel au moins l'une parmi la concentration de référence en BAIB et la concentration de référence en SDMA - si elle est mesurée - reflète le 95e percentile de la concentration en BAIB et en SDMA dans des échantillons provenant d'animaux en bonne santé.

11. Procédé selon la revendication 8, dans lequel l'anticorps comprend un marqueur.
